# EUROPEAN PATENT SPECIFICATION

(11) **EP 3 727 588 B1**
(45) Date of publication and mention of the grant of the patent: **23.08.2023**
(21) Application number: 18852722.0
(22) Date of filing: 21.12.2018
(51) Int. Cl.: A61P 25/28, C07K 14/47, A61K 38/16

(54) **ACTIVATOR PEPTIDES OF THE ENZYME ADAM 10 AND RELATED USES IN THE TREATMENT OF ALZHEIMER'S DISEASE**
AKTIVATOR-PEPTIDE DES ENZYMS ADAM 10 UND ENTSPRECHENDE VERWENDUNGEN ZUR BEHANDLUNG VON MORBUS ALZHEIMER
PEPTIDES ACTIVATEURS DE L'ENZYME ADAM 10 ET LEURS UTILISATIONS DANS LE TRAITEMENT DE LA MALADIE D'ALZHEIMER

(30) Priority: 22.12.2017 IT 201700149130
(43) Date of publication of application: 28.10.2020
(73) Proprietor: UNIVERSITA' DEGLI STUDI DI MILANO, 20122 Milano (IT)
(72) Inventor: MARCELLO, Elena, 20133 Milano (MI) (IT); DI LUCA, Monica Maria Grazia, 20145 Milano (MI) (IT); DI MARINO, Daniele, 22100 Como (CO) (IT); MUSARDO, Stefano, 1202 Geneve (CH); THERIN, Sebastien Michel, 38420 Domène (FR); GARDONI, Fabrizio, 20867 Caponago (MB) (IT)
(74) Representative: Calogero, Ida
(86) International application number: PCT/IB2018/060511
(87) International publication number: WO 2019/123413

(56) References cited:
- CN-A- 106 975 071
- ITTNER LARS M ET AL: "Dendritic Function of Tau Mediates Amyloid-[beta] Toxicity in Alzheimer's Disease Mouse Models", CELL, vol. 142, no. 3, 6 August 2010 (2010-08-06), pages 387-397, XP028931189, ISSN: 0092-8674, DOI: 10.1016/J.CELL.2010.06.036
- KRISTINA ENDRES ET AL: "Regulation of Alpha-Secretase ADAM10 In vitro and In vivo: Genetic, Epigenetic, and Protein-Based Mechanisms", FRONTIERS IN MOLECULAR NEUROSCIENCE, vol. 10, 17 March 2017 (2017-03-17), XP055498755, DOI: 10.3389/fnmol.2017.00056
- RAFAELA PERON ET AL: "Alpha-Secretase ADAM10 Regulation: Insights into Alzheimer's Disease Treatment", PHARMACEUTICALS, vol. 11, no. 1, 29 January 2018 (2018-01-29), page 12, XP055498765, DOI: 10.3390/ph11010012
- ELENA MARCELLO ET AL: "ADAM10 as a therapeutic target for brain diseases: from developmental disorders to Alzheimer's disease", EXPERT OPINION ON THERAPEUTIC TARGETS, vol. 21, no. 11, 3 October 2017 (2017-10-03), pages 1017-1026, XP055498779, UK ISSN: 1472-8222, DOI: 10.1080/14728222.2017.1386176
- ELENA MARCELLO ET AL: "Endocytosis of synaptic ADAM10 in neuronal plasticity and Alzheimer's disease", JOURNAL OF CLINICAL INVESTIGATION, vol. 123, no. 6, 1 June 2013 (2013-06-01), pages 2523-38-S1-S10, XP055499906, ISSN: 0021-9738, DOI: 10.1172/JCI65401
- E. MARCELLO ET AL: "Synapse-Associated Protein-97 Mediates ?-Secretase ADAM10 Trafficking and Promotes Its Activity", JOURNAL OF NEUROSCIENCE, vol. 27, no. 7, 14 February 2007 (2007-02-14), pages 1682-1691, XP055100159, ISSN: 0270-6474, DOI: 10.1523/JNEUROSCI.3439-06.2007

## Description

The present invention relates to new peptides that increase the activity of the enzyme ADAM10 by acting at various levels in the cascade of events that leads to the accumulation of the β-amyloid peptide. Pharmaceutical compositions are also contemplated comprising activator peptides of the enzyme ADAM10 and the related uses in the medical field for the treatment and prevention of the pathologies wherein an increase or accumulation of the β-amyloid peptide occurs, such as Alzheimer's disease, head injury and post-traumatic stress disorder.

Alzheimer's disease (AD) is a progressive neurodegenerative pathology characterized by memory and cognitive function disorders. Initially, slight short-term memory alterations appear, but as the pathology progresses the symptoms worsen until the loss of executive functions.

The immense social and economic effects of AD have led to the study of the mechanisms of the disease and the development of therapeutic agents for the treatment thereof. However, as yet there are no drugs available that can block the progression of the disease. Current therapeutic treatments temporarily relieve the symptoms without stopping the relentless progression of the disease.

The development of effective treatments was limited by the lack of knowledge of the mechanisms underpinning the disease. In fact, it is only possible to develop therapies able to effectively modify the progression of the disease when the molecular bases of the pathology are known.

In the case of AD, genetic and pathology-related evidence supports the hypothesis of amyloid, which indicates the β-amyloid peptide (Aβ) as the key element of the disease that can trigger a cascade of events that leads to synaptic dysfunction, tauopathy and neuronal loss [1]. Aβ derives from amyloid precursor protein (APP) thanks to the concentrated action of BACE1 and γ-secretase. Alternatively, APP can be cut from ADAM10 in the domain corresponding to Aβ. Therefore, the activity of ADAM10 prevents the formation of Aβ and generates the long neurotrophic fragment sAPPα.

The pathogenesis of AD is characterized by a cascade of events of a sequential nature, therefore acting on a therapeutic target located at the start of the cascade could lead to a therapy able to modify the course of the disease; on the contrary, acting on a target downstream may only improve the symptoms without acting on the progression [2].

In this context, the activation of ADAM10 could represent a potential therapeutic strategy with various advantages.

Firstly, ADAM10 competes with BACE1 to cut APP, therefore reinforcing the activity of ADAM10 significantly reduces the generation of Aβ, as shown in neuronal cultures [3] and in transgenic animal models of AD [4].

In the second place, the fragment sAPPα of APP derived from cutting ADAM10 has neurotrophic and neuroprotective properties. sAPPα is necessary for the maintenance of the dendritic integrity in the hippocampus [5]. Furthermore, sAPPα can interfere with the APP-dependent activation of the signalling pathway of JNK (JUN N-terminal kinase) and can therefore have an anti-apoptotic effect [6]. The effect on neurogenesis was demonstrated by the increased proliferation of neural precursors in the subventricular zone, a neurogenic niche, following the intraventricular injection of sAPPα [7]. Furthermore, the expression of ADAM10 reinforces neurogenesis in the adult hippocampus, which is reduced by mutations associated with late-onset AD [8]. A therapeutic approach that targets the overexpression of sAPPα in the brain thanks to the use of adeno-associated virus has explored the therapeutic potential of this strategy in the improvement of the pathological characteristics of AD in animals that already presented signs of the pathology and amyloidosis. The results indicate that sAPPα can mitigate cognitive and memory losses, influencing the long-term potentiation (LTP) of synapses and spine density in AD animals [9]. The sAPPα fragment also induces the recruitment of microglia and the activation of TREM2 and of insulin degrading enzyme (IDE), a protease produced by microglia which, as well as neprilysin [10], contributes to the clearance of Aβ [11], improving amyloidosis-related pathology.

Furthermore, the reduction of ADAM10 leads to an increase in the membrane levels of a substrate thereof (PrPC) [12], which has been shown to operate as a receptor and mediator of neurotoxicity in different proteinopathies such as AD. In fact, it has been demonstrated that PrPC is a neurone receptor of Aβ oligomers [13,14]. Therefore, an increase in the enzyme activity of ADAM10 would reduce the levels of this Aβ receptor limiting the toxic effects thereof at synaptic level. Furthermore, the metabolism of PrPC determines the formation of the neuroprotective fragment N1 which reduces the activation of the p53 pathway [15] and at extracellular level binds and neutralizes Aβ [16].

The main concern of the development of a targeted therapy for increasing the activity of ADAM10 relates to the large number of ADAM10 substrates that have been identified [17]. However, the increase in levels of ADAM10 and, consequently, the increase in the enzyme activity thereof, are surprisingly well tolerated both in mice [4,8] and in human beings [18].

Considering the high potential of ADAM10 as a therapeutic target for AD, we studied the cellular mechanisms responsible for the regulation of ADAM10 in the neurons. In this type of cells, ADAM10 is localized in the synapses, present in the presynaptic vesicles [19] and enriched in the postsynaptic density (PSD) of the excitatory synapses [20,21]. At post-translational level, the activity of ADAM10 at synaptic level can be controlled by the trafficking thereof as ADAM10 only cuts the substrates thereof when it is correctly inserted in the plasma membrane [20,22]. The postsynaptic levels of ADAM10 are regulated by the interaction of the cytoplasmic tail thereof with other proteins, such as synapse-associated protein 97 (SAP97) and AP2 clathrin adaptor complex [20,23]. AP2 is a heterotetrameric complex of 4 subunits (α-adaptin, β2-adaptin, µ2 and σ) which binds specific sequences of endocytosis in membrane proteins. SAP97 mediates the local trafficking of ADAM10, as it binds the Golgi outpost enzyme at dendritic level and mediates the transport thereof until the PSD [24], while the interaction with AP2 induces the endocytosis of ADAM10 by the synaptic membranes [23]. These mechanisms that regulate the localization of ADAM10 are altered in AD patients. In particular, a reduction of the ADAM10/SAP97 interaction and a concomitant increase in the ADAM10/AP2 association were observed in the hippocampus of AD patients compared with control subjects [24,25]. These alterations lead to a reduction in the activity and synaptic localization of ADAM10 in AD patients. These data indicate that there is a defect in the mechanisms that regulate the synaptic localization of ADAM10 and that this can be considered a pathological feature of AD synapsis. Furthermore, these altered mechanisms in AD constitute a potential pharmacological target positioned at the beginning of the AD pathogenesis cascade. Peptides are instruments that can interfere in a specific way in protein-protein interactions (PPI), both *in vitro* and *in vivo,* and are therefore effective tools for manipulating biological pathways.

The authors of the present invention have now developed peptides characterized by high cell permeability that can interfere specifically with the ADAM10/AP2 interaction, which is increased in AD patients, reducing the endocytosis of the enzyme, and stabilizing the localization of ADAM10 in synapsis, therefore restoring the physiological activity thereof.

In this way it is possible to increase the levels and activity of ADAM10 in synapsis to limit the generation of Aβ.

The peptides are comprised of a peptide derived from the HIV TAT protein, TAT48-57, which relocates through the plasma membranes, and enables the transport of conjugated peptides known as effectors. The use of this type of peptides (known as cell permeable peptides or CPPs) has already been exploited in clinical studies for acute neurological diseases [26].

In the study of Ittner L. M. et al. [42] the treatment of an experimental murine model of Alzheimer (APP23) with the peptide Tat-NR2B9c inhibitor of the NR/PSD-9 interaction, whose sequence comprises the aminoacid of the carboxy end of NR2b fused to a peptide HIV 1-Tat to enhance the permeability through plasmatic membrane has been diosclosed. By administration of the peptide YGRKKRRQRRRKLSSIESDV to APP23 mice an in vitro and in vivo protective effect on neuronal cells has been detected; the perburbation of the NR/PSD-95 interaction is sufficient to prevent mortality and memory deficit in APP23 mice.

Therefore, the subject matter of the present invention relates to a peptide characterized by an amino acid sequence having formula (I):

YGRKKRRQRRRGGSGX₁PPX₂X₃X₄PRX₅ (I)

wherein:
X₁ and X₅ each independent from the other, identical to or different from each other, are selected from the group consisting of L-amino acids characterized by apolar or polar neutral side chains;
X₂ and X₄ each independent from the other, identical to or different from each other, are arginine or lysine;
X₃ is glutamine or asparagine;
for use in the medical field.

In particular, the RQR triplet represents the fulcrum region for inhibitory activity. On the basis of these considerations, peptides with the following variations of the RQR triplet can be considered equivalent to the original peptides: KQK, RQK, KQR, RNR, KNR, RNK.

According to a preferred embodiment of the present invention, said L-amino acids are characterized by apolar neutral side chains and are selected from the group consisting of glycine, leucine, isoleucine, alanine, phenylalanine, methionine, proline, tryptophane, and valine. Again, according to a preferred embodiment of the present invention, said L-amino acids are characterized by polar neutral side chains and are selected from the group consisting of glutamine, asparagine, serine, tyrosine, threonine and cysteine.

In a particularly preferred embodiment X₁ is glutamine or leucine and X₂ is arginine or lysine.

Preferably, the peptide for use in medical field according to the present invention comprises or consists of the amino acid sequence YGRKKRRQRRRGGSGQPPRQRPRG (SEQ ID NO: 1).

Again, preferably the peptide for use in medical field according to the present invention comprises or consists of the amino acid sequence YGRKKRRQRRRGGSGLPPRQRPRL (SEQ ID NO:2).

According to further alternative embodiments of the present invention the peptide for use in the medical field comprises or consists of the amino acid sequence YGRKKRRQRRRGGSGQPPRQRPRE (SEQ ID NO: 3).

The present invention further relates to a peptide as defined above in for use the prevention and/or treatment of pathologies caused by an increase or accumulation of β-amyloid peptide. Preferably, said pathology caused by an increase of β-amyloid peptide is Alzheimer's disease. On the other hand in situation such as head injury, post-traumatic stress disorder, the increase of β-amyloid peptide is a consequence of trauma or stress.

The term prevention is used here with reference to acute events such as head injury, where treatment with the peptide according to the invention could prevent the increase in amyloid that is observed and that could be the cause of an increased risk of dementia in subjects at risk, for example boxers.

The peptides according to the invention can be prepared through solid-phase peptide synthesis based on Fmoc chemistry or through the expression of the recombinant pathway. In particular, the nucleotide sequence for the expression of the peptide having SEQ ID NO: 1 is as follows: 5'-tatggtcgtaagaagcgccgccaacgacgtcgaggcggcagcggacagccccctcgtcagcgcccgcgg ggt -3' (SEQ ID NO:4).

Whereas for the peptide having SEQ ID NO:2 the following nucleotide sequence can be used: 5'-tatggtcgtaagaagcgccgccaacgacgtcgaggcggcagcggacttccccctcgtcaacgccctcgtctc-3' (SEQ ID NO:8).

The present invention also contemplates the mimetic-peptide variants of said peptides, i.e. molecules that while maintaining the structure and bioactivity of the original molecule display greater stability (provided by improved resistance to proteolysis and/or physical and chemical degradation). These variants derive from the incorporation of amino acid residues not found in nature (D-amino acids) in the place of L-amino acids and/or non-proteinogenic amino acids. The amino acids in proteins are all in L configuration but it has been demonstrated that peptides synthesized with amino acids in D configuration show greater protection against the proteolitic activity of endogenous proteases and therefore a longer half-life. For this reason the synthesis of peptides according to the invention with amino acids in D configuration can be considered a valid and functioning variation of the original sequences of peptides.

The present invention further relates to a pharmaceutical composition comprising at least one peptide as defined above, as the active ingredient, together with one or more pharmacologically acceptable excipients and/or adjuvants.

Preferably said pharmaceutical composition is formulated to be adapted for systemic administration through intravenous infusion.

According to an alternative embodiment of the present invention, the pharmaceutical composition according to the invention is adapted for oral or intranasal administration.

Further subject matter of the present invention is also a pharmaceutical composition for use in the prevention and/or treatment of pathologies in which there is an increase or accumulation of β-amyloid peptide, such as in the Alzheimer's disease. The invention finally relates to the use of one of the peptides as defined above as an ADAM10 endocytosis inhibitor in basic research applications for studying the effect of this mechanism on the cutting of the substrates thereof (e.g. CD44 [27]). The present invention will now be described, for non-limiting illustrative purposes, according to a preferred embodiment thereof, with particular reference to the attached figures, wherein:

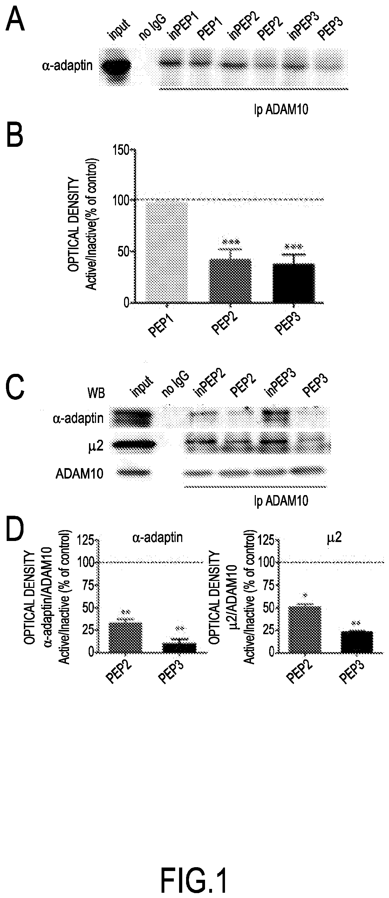

- Figure 1 shows the reduction of the interaction between ADAM10/AP2 following treatment with the peptides PEP3 (SEQ ID NO:1) and PEP2 (SEQ ID NO:2). (A) Samples of homogenate obtained from acute hippocampal slices treated both with active and inactive CPPs (10 µM, 30 min), were immunoprecipitated with the antibody anti-ADAM10 to evaluate the interaction levels with the α-adaptin subunit of AP2. (B) Quantitative analyses of the experiments performed in A. The optical density (OD) and the analysis of the PEP/inPEP ratio shows that PEP2 and PEP3 significantly reduce the formation of the ADAM10/AP2 complex (PEP2/inPEP2 41.60 ± 23.86% ***p = 0.0054, PEP3/inPEP3 37.40 ± 21.76% ***p = 0.003). (C) Primary cultures of neurons were treated with PEP2 or PEP3 (1µM, 30 min) or with the corresponding control peptide thereof; the cell lysates were immunoprecipitated with the antibody anti-ADAM10 and the interaction with the subunits of the complex AP2, α-adaptin and µ2 was evaluated. (D) Quantitative analysis of the experiments in C. The OD shows that PEP2 and PEP3 significantly reduce the levels of the ADAM10/AP2 complex (α-adaptin PEP2/inPEP2 33.92 ± 5,0% **p = 0.0019; PEP3/inPEP3 10.84 ± 5.61% **p = 0.0013; µ2 PEP2/inPEP2 52.01 ± 3.77% **p = 0.002; PEP3/inPEP3 22.43 ± 1.25% ***p <0.0001).

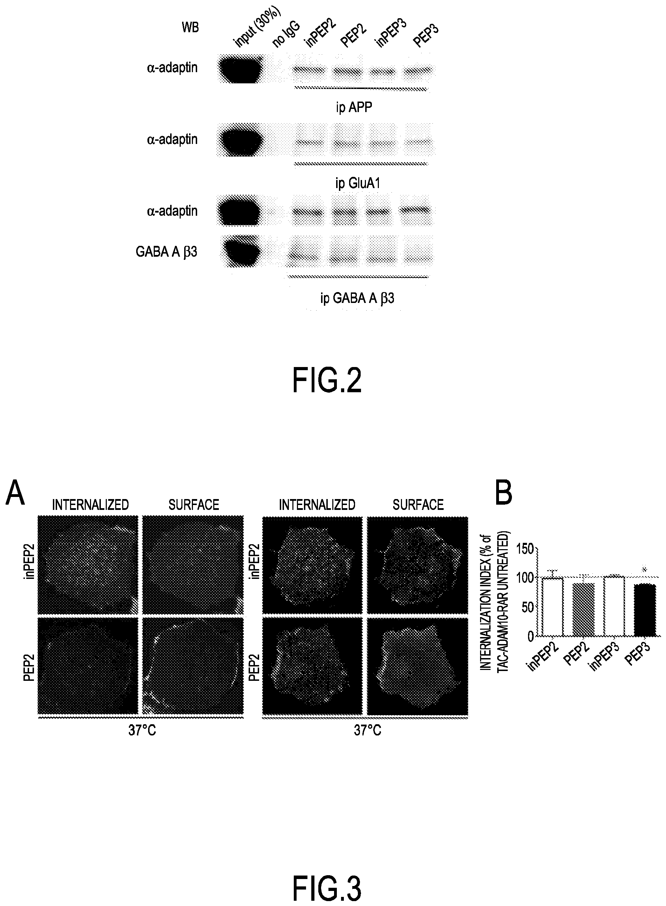

Figure 2 shows the results that demonstrate how the peptides PEP2 (SEQ ID NO:2) and PEP3 (SEQ ID NO:1) do not alter the interaction between AP2 and other target proteins. Samples of acute hippocampal slices treated with PEP2 and PEP3 were immunoprecipitated with an anti-APP, anti-GluA1 or anti-GABA-A β3 antibody and the levels of interaction with the α-adaptin subunit were evaluated. As shown in the representative immunoblots, neither PEP2 nor PEP3 interfere with the bond between the AP2 complex and the different proteins analysed.
Figure 3 shows the results that illustrate how the endocytosis of ADAM10 is modified by treatment with the peptides of the invention CPP. (A) The antibody uptake test was performed in COS7 cells transfected with TacADAM10-RAR, and the internalization was analysed following treatment with the active peptides PEP2 (SEQ ID NO:2) or PEP3 (SEQ ID NO:1) or with the respective inactive peptides thereof (1 µM, 30 min). Representative image of cells brought back to 37°C to allow endocytosis. A decrease in the number of internalization puncta of TacADAM10-RAR was observed following treatment with the active peptides. (B) Quantization of the internalization index of the experiment in A. Treatment with PEP3 causes a significant reduction of the internalization index, while treatment with PEP2 does not affect endocytosis (TacADAM10-RAR PEP2 =-10.95 ± 15.82%, TacADAM10-RAR inPEP2 = -1.84 ± 13.92%; TacADAM10-RAR PEP3 = -12.85 ± 1.61%, TacADAM10-RAR inPEP3 = +1.30 ± 2.81% *p=0.0489)

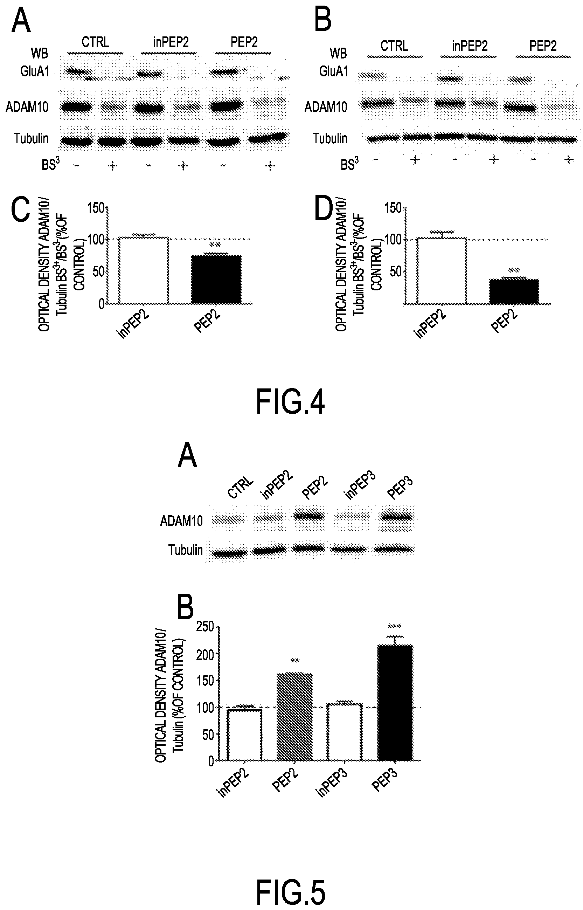

Figure 4 shows the results of the modulation of the localization in membrane of ADAM10 following treatment with the peptides of the invention. Immunoblot of ADAM10, GluA1 and of tubulin performed in acute hippocampal slices of rats incubated with the peptides PEP2 (SEQ ID NO:2) or PEP3 (SEQ ID NO:1) treated or not treated with the cross-linking agent BS³. Both of the peptides cause a significant reduction of the intracellular pool of ADAM10, which indicates an increase of the surface expression of ADAM10. Quantization of the experiment PEP2 74.22 ± 3.83% *p=0.009; inPEP2 102.90 ± 4.67% p>0.05; PEP3 37.52 ± 3.38% *p=0.0032; inPEP3 102.70 ± 9.73% p>0.05).
Figure 5 shows the results related to the modulation of the synaptic localization of ADAM10 following treatment with the peptides of the invention CPP. (A) Western Blot analysis of the levels of ADAM10 present in the synaptic fraction of primary hippocampal neurons treated with the peptides PEP2 (SEQ ID NO:2) and PEP3 (SEQ ID NO:1) or the corresponding inactive peptides thereof. (B) Quantitative analysis of the experiments reported in (A). Both of the treatments performed with the active peptides cause an increase in the synaptic localization of ADAM10 with respect to the controls (PEP2 +65.75 ± 3.19%, inPEP2 -5.55 ± 7.72% **p<0.01 PEP2 vs inPEP2, PEP3 +114.90 ± 17.89%, inPEP3 +5.30 ± 4.93% ***p<0.001, PEP3 vs in PEP3).

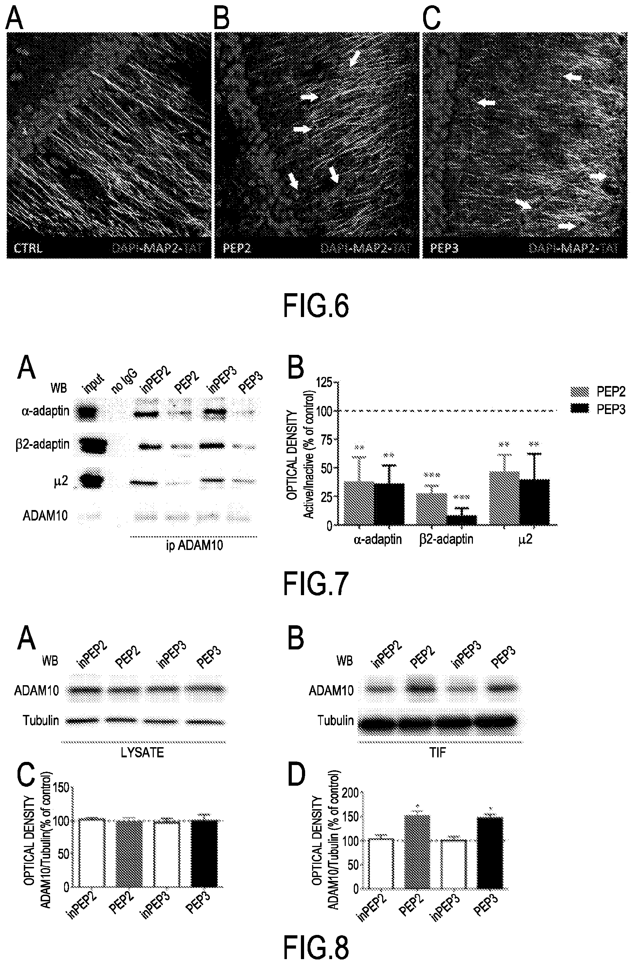

Figure 6 shows the results related to the capacity of the peptides according to the invention to cross the blood-brain barrier (BBB). Mice C57BL/6 six weeks old were treated with the active and inactive peptides through intraperitoneal injection (3 nmoles/g) or with saline solution (control mice). 24 hours after treatment the animals were perfused with paraformaldehyde and an immunohistochemistry test was performed on the coronal sections of brain obtained from control mice (A) and from mice treated with PEP2 (B) and PEP3 (C). The coronal sections were incubated with the nuclear marker DAPI, with the neuronal marker MAP2 and with the direct antibody against the N-terminal domain of TAT. As shown by the arrows in images B and C, the presence of a specific signal of the anti-TAT antibody can be identified which indicates that both PEP2 and PEP3 can cross the BBB and penetrate into the neuron cells. On the contrary in the control mice treated with saline, there is no visible presence of such signal.
Figure 7 shows the results related to the reduction of the association between ADAM10/AP2 following treatment with the peptides PEP2 (SEQ ID NO:2) and PEP3 (SEQ ID NO:1) *in vivo.* (A) The brain homogenate of control mice and treated mice was immunoprecipitated with the antibody anti-ADAM10 (rabbit) and through Western Blot the co-immunoprecipitation of the α-adaptin, β2-adaptin and µ2 subunits was evaluated. The immunoprecipitation of ADAM10 was detected with an anti-ADAM10 antibody produced in rats. (B) Quantitative analysis of the data obtained in A. Both of the peptides PEP2 and PEP3 report a significant reduction of the co-precipitation of the different subunits of AP2 (PEP2 α-adaptin - 62.82 ± 12.78% **p< 0.01, β2-adaptin -73.36 ± 4.23% ***p<0.001, µ2 -54.39 ± 7.88% **p<0.01; PEP3 α-adaptin -64.96 ± 9.81 **p< 0.01, β2-adaptin -92.45 ± 4.02% ***p<0.001, µ2 -61.06 ± 11.63% **p<0.01).
Figure 8 shows the results related to the modulation of the synaptic localization of ADAM10 following treatment with CPP *in vivo.* C57BL/6 mice were treated with both active peptides and after 24 hours Western Blot analyses were performed on total lysate samples (panel A), or on synaptic fraction samples (TIF) (panel B). (C) Quantitative analysis of the experiments in A, expressed as optical density (OD) of the ADAM10/Tubulin ratio. Treatment with CPPs does not alter the levels of ADAM10 present in the total brain lysate (inPEP2 102.00 ± 5.24%, PEP2 98.48 ± 11.02% p>0.05, inPEP3 97.53 ±10.85%, PEP3 100.00 ±11.62% p>0.05). (D) Quantitative analysis of the experiments performed in B, expressed as ADAM10/Tubulin OD ratio. Both of the active peptides increase the synaptic localization of ADAM10 (inPEP2 103.60 ± 8.69%, PEP2 151.70 ± 9.77% *p<0.05, inPEP3 100.20 ± 8.22%, PEP3 147.50 ± 7.09% *p<0.05).

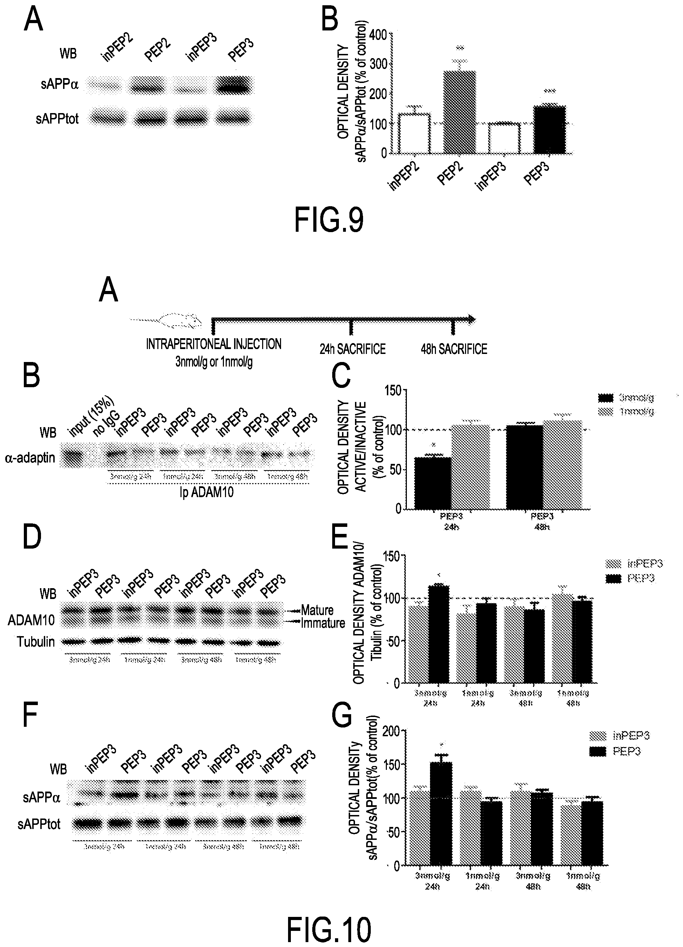

Figure 9 shows the results that demonstrate how treatment with active peptides positively modulates the activity of ADAM10 *in vivo.* The Western Blotting analyses were performed using the protein extract of the soluble brain fraction of mice treated with the peptides PEP2 or PEP3. (B) Quantitative analysis of the data obtained in (A) expressed as optical density of the ratio between total soluble APP protein, and the soluble alpha fragment APP (sAPPα). The ratio is increased in mice treated with both of the active peptides (inPEP2 130.80 ± 34.66%, PEP2 271.40 ± 36.90% **p<0.05, inPEP3 98.41 ± 9.69%, PEP3 155.50 ± 15.05% ***p<0.05).
Figure 10 shows the results of the tests for evaluating the times and effective dose of the intraperitoneal injection. (A) Experimental paradigm. (B) Brain homogenates deriving from control mice or mice treated with 1 nmol/g or 3 nmol/g, and sacrificed after 24 hours or 48 hours, were immunoprecipitated with the antibody for ADAM10 and the co-precipitation with α-adaptin was evaluated. (C) Quantization of the PEP3/inPEP3 OD of the experiment depicted in B. PEP3 significantly reduces the ADAM10/AP2 co-precipitation only if administered at the dose of 3 nmol/g and the effect only persists for 24 hours (inPEP3/PEP3 -35.74 ± 7.80%, *p<0.05). (D) TIF fraction obtained from mice treated with PEP3 or inPEP3 at the dose of 1 nmol/g or 3 nmol/g, and sacrificed 24 hours or 48 hours later. The TIF fraction was used to determine the synaptic levels of ADAM10. (E) Quantization of the experiment in D, ADAM10/tubulin OD ratio. Only mice treated with the dose of 3 nmol/g and sacrificed 24 hours after the injection, display a significant increase in the synaptic levels of ADAM10 (inPEP3 90.20% ± 5.39%, PEP3 113.70% ± 2.83% *p<0.05). (F) The analyses through the Western Blotting technique were performed in the soluble brain fraction obtained from mice treated with PEP3 at the dose of 1 nmol/g or 3 nmol/g and sacrificed 24 hours or 48 hours after administration. The representative immunoblot images, which were obtained using antibodies for sAPPα and for the total form of sAPP, display an increase in the release of sAPPα exclusively in animals treated with PEP3 at the dose of 3 nmol/g and analysed 24 hours after injection. (G) Quantization of the experiment in F. The OD of the ratio between sAPPα and sAPP-total was measured in each experimental group (3 nmol/g-24 hours inPEP3 108.70 ± 8.00%, PEP3 151.60 ± 12.22% *p<0.05).

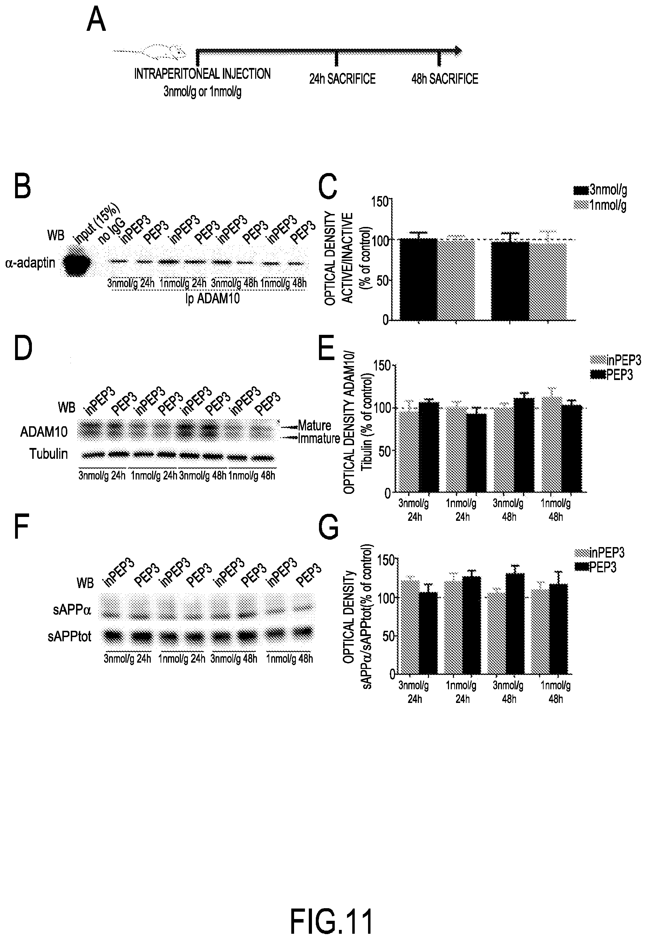

Figure 11 shows the results of the test for evaluating the efficacy of the subcutaneous injection of the peptides of the invention. (A) Experimental paradigm. (B) Brain homogenates deriving from mice treated through sub-cutaneous injection, with the dose of 1 nmol/g or 3 nmol/g, and sacrificed after 24 hours or 48 hours, were immunoprecipitated with the antibody for ADAM10 and the co-precipitation with α-adaptin was evaluated. (C) Quantization of the PEP3/inPEP3 OD of the experiment reported in B. The sub-cutaneous injection does not modify the interaction between ADAM10 and the AP2 complex. (D) Representative image of the immunoblot of ADAM10 through Western Blotting analysis in the TIF fraction. (E) Quantization of the experiment in D, ADAM10/tubulin OD ratio. PEP3, administered through sub-cutaneous injection, does not modify the synaptic availability of ADAM10. (F) The analyses performed through Western Blotting were performed in the soluble brain fraction for the purpose of measuring the release of sAPPα. (G) Quantization of the experiment in F. The ratio of the OD between sAPPα and sAPP-total was measured for each experimental group; the sub-cutaneous injection of PEP3 does not interfere with the release of sAPPα.

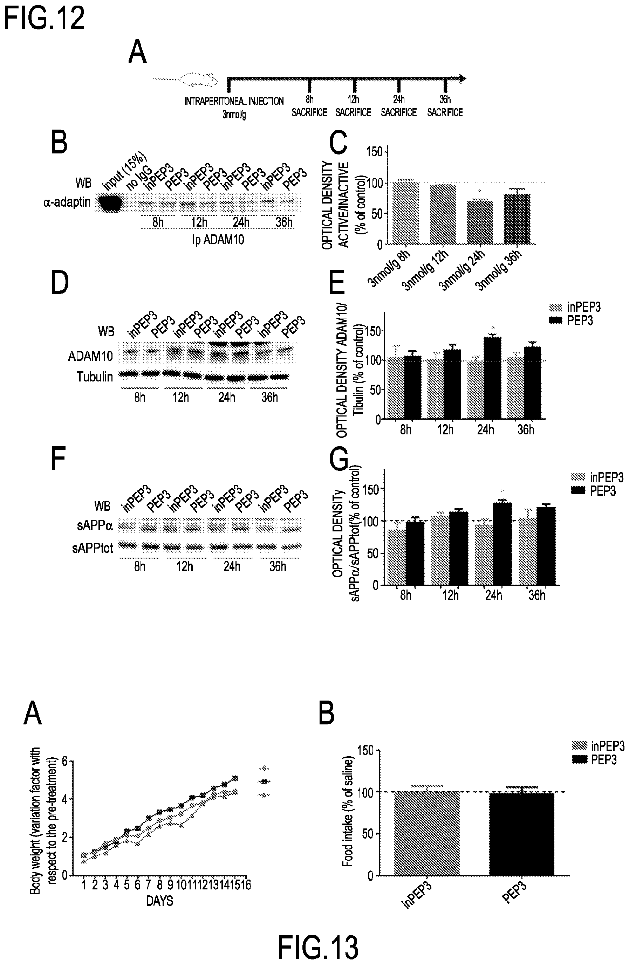

Figure 12 shows the results of the test for evaluating the efficacy of PEP3 over time. (A) Experimental paradigm. (B) Brain homogenates obtained from mice treated with intraperitoneal injection of PEP3 (3 nmol/g), and sacrificed after 8, 12, 24 or 36 hours, were immunoprecipitated with the antibody for ADAM10 and the co-precipitation with α-adaptin was evaluated. (C) Quantization of the PEP3/inPEP3 OD of the experiment reported in B. PEP3 significantly reduces the ADAM10/AP2 co-precipitation only 24 hours after administration (PEP3/inPEP3 -31.00 ± 6.13%, *p<0.05). 36 hours after injection a slight reduction of the ADAM10/ α-adaptin interaction can be observed, although it is not statistically significant (PEP3/inPEP3 -19.69 ± 16.86%). (D) Representative image of the immunoblot of ADAM10 through Western Blotting analysis. (E) Quantization of the experiment in D, ADAM10/tubulin OD ratio. Only the animals that were sacrificed after 24 hours displayed a significant increase in the synaptic levels of ADAM10 (inPEP3 6.43 ± 9.23%, PEP3 137.40 ± 6.32% *p<0.05). (F) The representative image of the immunoblot, obtained using antibodies for sAPPα and for the total form of sAPP, displays an increase of the release of sAPPα only in the mice that were sacrificed after 24 hours. (G) Quantization of the experiment in F, ratio of the OD between sAPPα and sAPP-total (24 hours inPEP3 92.84 ± 10.05%, PEP3 126.30 ± 6.23% *p<0.05).
Figure 13 shows the data related to the body weight and food intake of the animals. (A) The body weight of each animal was noted on a daily basis before performing the injection. The graph shows the mean of the daily weights of each group. As can be seen from the image, treatment with CPP does not determine a reduction of the body weight, or a difference between the different groups. (B) To determine the amount of food taken by the different experimental groups we calculated the ratio between the daily food intake, measured by weighing the food in the cage, and the mean body weight of the animals present inside it. As shown by the histogram, no alterations are observed in the food intake between animals treated with the inactive and the active peptide (inPEP3 99.03±2.03%, PEP3 97.09±2.18% p>0.05).

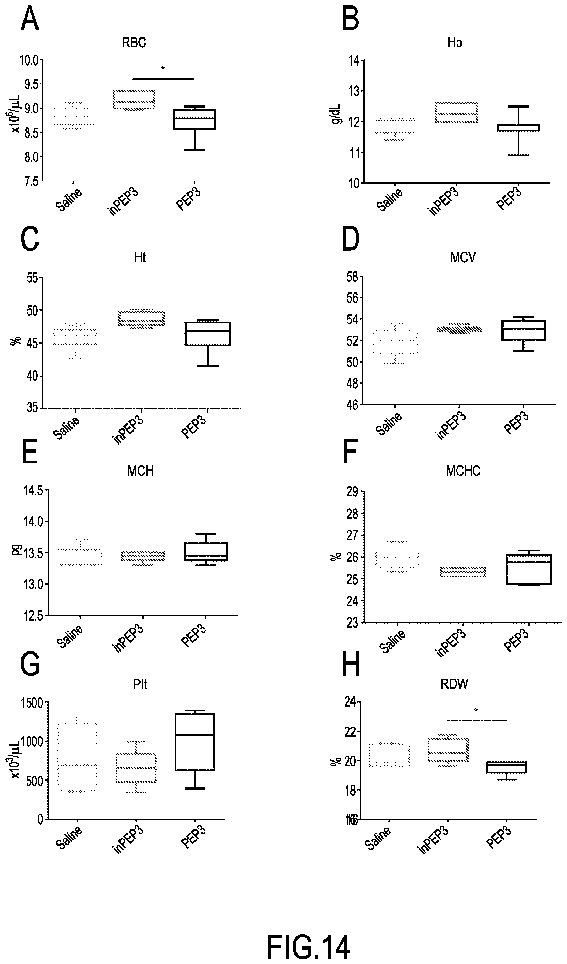

Figure 14 shows the graphic distribution of the data related to the blood count reported in Table 1 and the corresponding statistical analyses. For all the markers considered, except the MCH, the mean of the measured values falls within the reference interval without undergoing significant variations. Only the number of red blood cells (RBC) (A) and the red cell distribution width (RDW) measured in the animals treated with PEP3 (H) is higher with respect to the other groups, however these differences do not have any biological relevance.

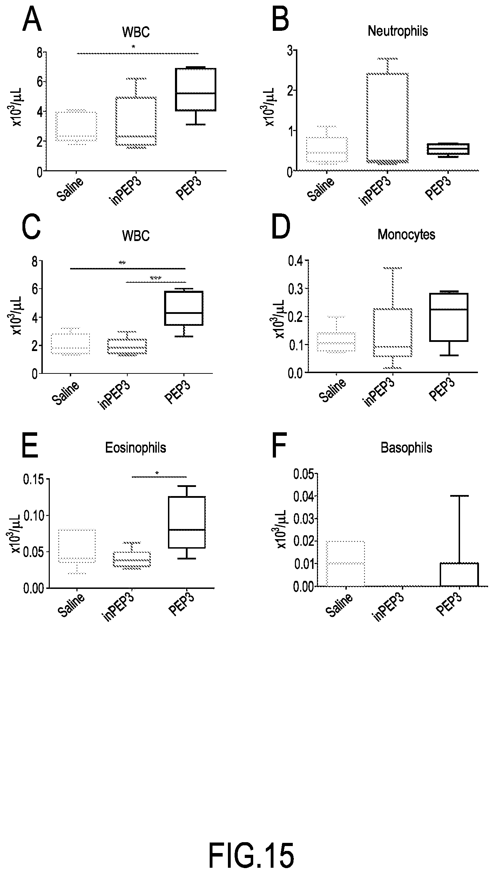

Figure 15 shows the graphic distribution of the data related to the leukogram reported in Table 2 and the corresponding statistical analyses. The values of the total white blood cells and differentials are lower in all mice with respect to the reference intervals, with the exception of the eosinophils and basophils. The mice treated with the active peptide display statistically higher values in the white blood cell WBC (A), lymphocyte (C) and eosinophil (E) count with respect to mice treated with saline and/or with inPEP3 (*p<0.05, **p<0.01, ***p<0.001).

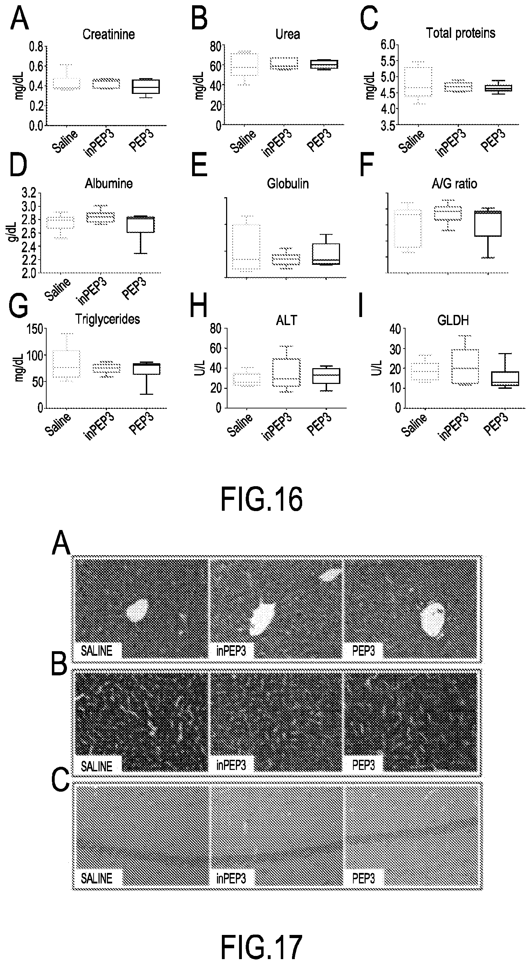

Figure 16 shows the graphical representation of the data reported in Table 3. The statistical analyses do not show any significant changes between the different experimental groups, suggesting that the treatment does not induce any dysmetabolic phenomena.
Figure 17 shows the stains of liver and brain samples of all the animals fixed in formalin and included in paraffin. The haematoxylin-eosin stain does not show any structural alterations or lesions in the CA1 region of the hippocampus (C) or in the liver (A). In particular a moderate presence of vacuoles was observed inside the cytoplasm of the hepatocytes. However, such vacuoles do not shift the localization of the nucleus in the PAS-positive cells (B).

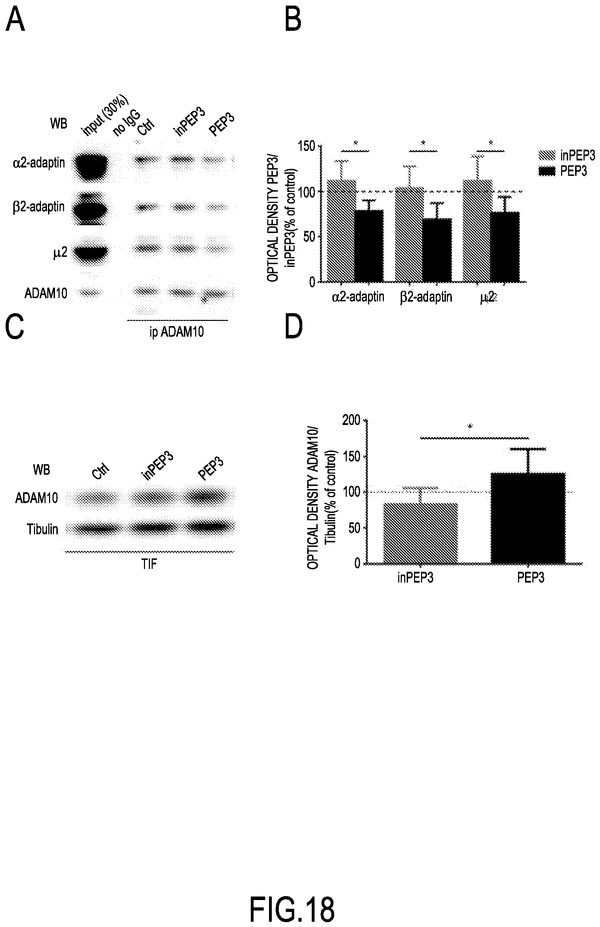

Figure 18 shows the effect of PEP3 on the formation of the ADAM10/AP2 complex and in the synaptic localization of ADAM10 following 14 days of treatment. (A) Samples coming from the total brain homogenate were immunoprecipitated with anti-ADAM10 antibodies; the co-immunoprecipitation of the AP2 subunits: α-adaptin, β2-adaptin and µ2 was evaluated. (B) Quantitative analysis of the OD obtained from the experiments in A. Treatment with PEP3 significantly reduces the interaction levels between ADAM10 and all the subunits of the AP2 complex (α-adaptin PEP3 -21.49 ± 5.20% inPEP3 +11.40 ± 9.86%; β2-adaptin PEP3 -30.77 ± 7.34% inPEP3 +3.70 ± 10.81%; µ2 PEP3 -23.60 ± 6.99% inPEP3 +11.80 ± 12.18%; *p<0.05). (C) Western Blot image of the levels of ADAM10 in the TIF. (D) Quantitative analysis of the experiments in C. The ratio between ADAM10/tubulin shows a significant increase of the synaptic levels of ADAM10 in animals treated with the active peptide (inPEP3 82.97 ± 10.18%, PEP3 125.60 ± 14.10% *p<0.05).

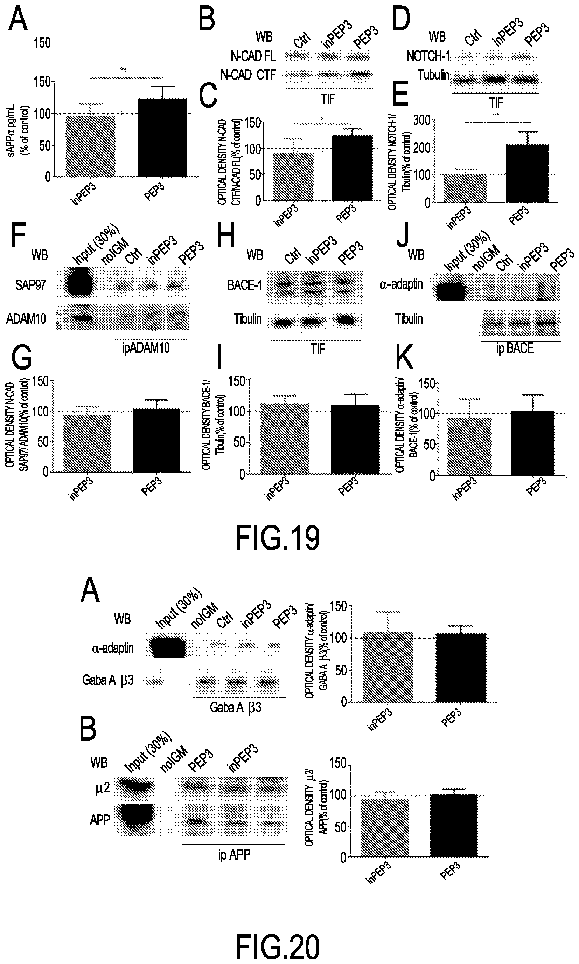

Figure 19 shows how the peptide of the invention PEP3 (SEQ ID NO:1) increases the enzyme activity of ADAM10 following 14 days' treatment. (A) The ELISA test performed on brain homogenate shows the increased release of sAPPα after treatment with PEP3 (inPEP3 94.93±5.62%, PEP3 121.50 ±5.99% **<0.01). (B) Western Blot representing the N-Cadherin levels. An antibody against the C-terminal portion of N-Cadherin was used to detect the full-length protein (N-CAD FL) and the C-terminal fragment (N-CAD CTF). The quantitative analysis (C) expressed as N-CAD CTF and full-length protein N-CAD FL ratio displays a significant increase in the cut of N-Cadherin after treatment with active peptide (inPEP3 90.22 ± 11.73%, PEP3 124.00 ± 5.42%, *p<0.05). In the same way, the Western Blot in D and the related quantitative analysis in E, show an increase in the production of the fragment of NOTCH-1 in mice treated with PEP3 (inPEP3 101.70 ± 9.45%, PEP3 207.60 ± 23.58%, **p<0.01). (F) Co-immunoprecipitation performed on total brain homogenate using an anti-ADAM10 antibody and detecting SAP97. The quantitative analysis (G) shows that treatment with PEP3 does not change the interaction between ADAM10 and SAP97 (inPEP3 -6.80 ± 5.86%, PEP3 +3.30 ± 6.40%). In the same way the Western Blot in (H) and (J) and the quantitative analyses in (I) and (K) show that treatment with PEP3 does not alter respectively either the synaptic localization of BACE-1 (inPEP3 111.00 ± 5.62%, PEP3 108.90 ± 7.24%), or the interaction between BACE-1 and α-adaptin (inPEP3 - 8.33 ± 13.13%, PEP3 +2.90 ± 11.16%).
Figure 20 shows the specific interference of the peptide PEP3 (SEQ ID NO:1) with the complex ADAM10/AP2. (A) Immunoprecipitation with the antibody anti-GABA-A β3 to detect the presence of α-adaptin. As shown in the histogram (right panel) the treatment with PEP3 does not modify the formation of the complex AP2/GABA-A β3 (inPEP3 +7.80 ±13.08%, PEP3 +6.00 ± 5.36%). In the same way, the co-IP analysis performed using an anti-APP antibody (B) shows that PEP3 does not alter the interaction between APP and the complex AP2.

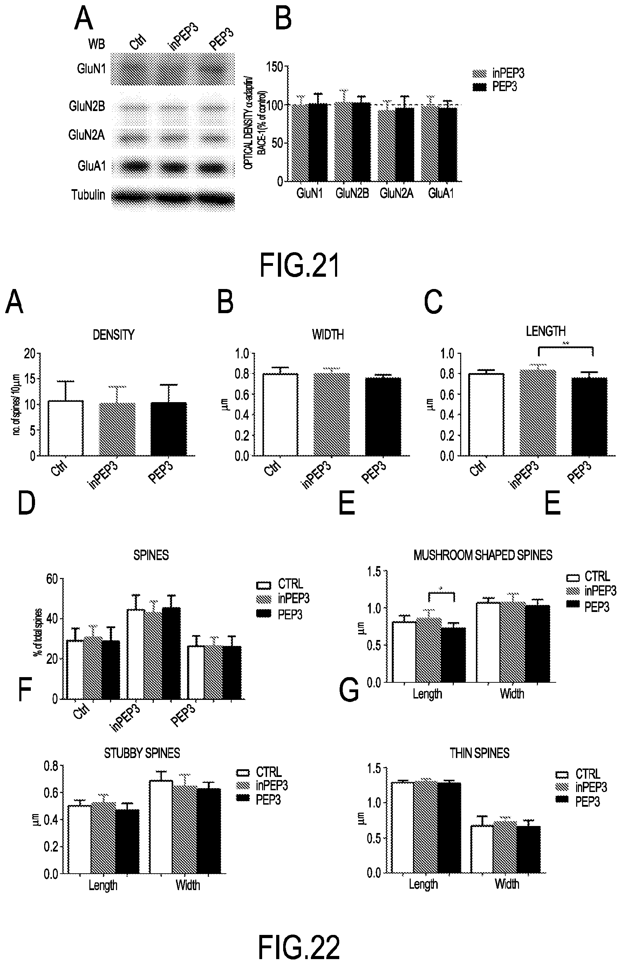

Figure 21 shows the Western Blot analyses that demonstrate how treatment with the peptide PEP3 (SEQ ID NO:1) does not alter the glutamatergic synapse composition. (A) Western Blotting analysis, performed for each sample, using anti-GluN1, anti-GluN2B, anti-GluN2A and anti-GluA1 and anti-tubulin antibodies. (B) Quantitative analysis of the images in (A) shows that treatment with the active peptide does not alter the receptor composition at synaptic level.
Figure 22 shows the analysis of the morphology and density of dendritic spines following 14 days' treatment with the peptide PEP3 (SEQ ID NO:1). For each group the number of dendritic spines at hippocampus level was counted. The graph in A shows that the treatment for PEP3 does not change the density of the dendritic spines (saline 10.68±1.19, inPEP3 10.15±1.05, PEP3 10.30±1.11, n. of spines/10µm). As shown in the graph B, changes in the width of the head of spines after treatment with PEP3 (saline 0.79±0.01 µm, inPEP3 0.79±0.02 µm, PEP3 0.75±0.01 µm) are not significant, whereas the length of the spines decreases in mice treated with the active peptide (C) (saline 0,80±0.01 µm, inPEP3 0.83±0.02 µm, PEP3 0.75±0.02 µm **p<0.01). For each experimental group no alterations were detected in the percentage of mushroom shaped, stubby and thin spines (D). (Mushroom shaped spines: saline 29.14 ± 1.91%, inPEP3 30.81 ± 1.78%, PEP3 28.83 ± 2.18%; stubby spines: saline 44.49 ± 2.28%, inPEP3 42.78 ± 1.86%, PEP3 45.25 ± 2.03%; thin spines: saline 26.37 ± 1.63%, inPEP3 26.41 ± 1.42%, PEP3 25.92 ± 1.68%). On the contrary, the analysis of the length and width of the spines (E-F-G) revealed a reduction of the length of mushroom shaped spines in mice treated with active peptide (saline 0.80±0.03, inPEP3 0.85±0.04, PEP3 0.73±0.02 *p<0.05).

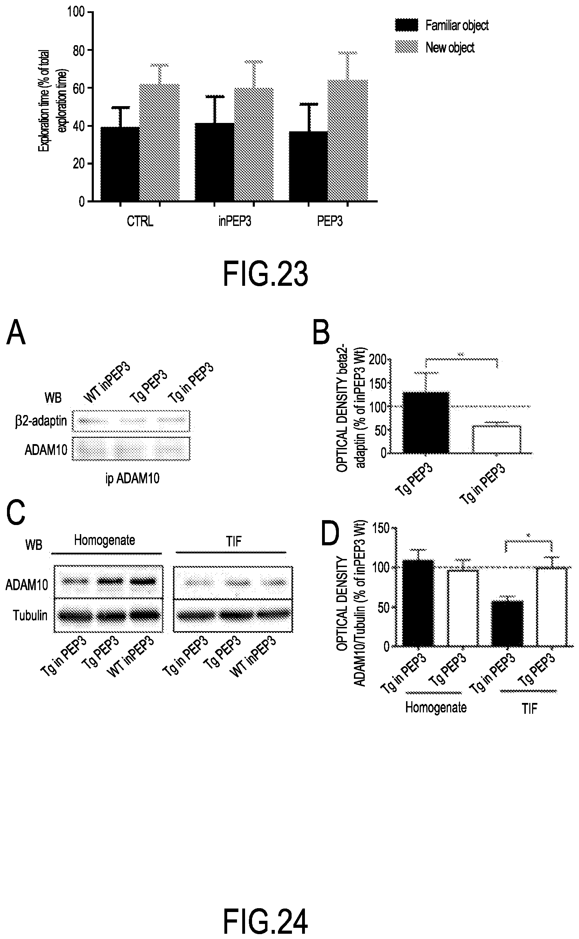

Figure 23 shows the results of the NORT test performed on animals treated with the active peptide PEP3 (SEQ ID NO:1) or the inactive peptide inPEP3 (SEQ ID NO:5) or with saline solution. The graph shows the percentage of time spent by the different experimental groups exploring the familiar object and the new object. The results obtained show that neither PEP3 nor inPEP3 lead to significant changes in time exploring the new object, with respect to animals treated with saline solution (saline 61.26 ± 3.57%, inPEP3 59.24 ± 4.58%, PEP3 63.52 ± 4.69%).
Figure 24 shows the results of the treatment of APP/PS1 mice of 9 months for 14 days with the active peptide PEP3 (SEQ ID NO:1) or the inactive peptide inPEP3 (SEQ ID NO:5) which restores the ADAM10 levels in synapsis. Panels A and B. Data related to the immunoprecipitation of ADAM10/β2 adaptin in wild-type (WT) and APP/PS1 mice treated for 14 days with inPEP3 (Tg inPEP3) or with PEP3 (Tg PEP3). Samples coming from the total brain homogenate were immunoprecipitated with anti-ADAM10 antibodies, the co-immunoprecipitation of the AP2/β2-adaptin subunit was evaluated. A significant reduction of the ADAM10/AP2 interaction was observed in APP/PS1 mice treated with PEP3-active compared with APP/PS1 mice treated with inPEP3-inactive (Tg inPEP3 131.70 ± 39.93%, Tg PEP3 57.59 ± 7.83%, ** p<0.05; the data were shown as a percentage of the values of WT mice treated with inPEP3). C. and D. Data related to the levels of ADAM10 obtained through Western Blotting analysis of samples of total homogenate and TIF obtained from WT and APP/PS1 mice treated for 14 days with PEP3-inactive (Tg inPEP3) or with PEP3 (Tg PEP3). Significant increase in the synaptic levels of ADAM10 in APP/PS1 mice treated with PEP3-active and compared with APP/PS1 mice treated with inPEP3 (Tg inPEP3 57.12 ± 6.65%, Tg PEP3 99.13 ± 13.64, *p<0.05). No modification to the total levels of ADAM10 in the different groups of animals was observed (Tg inPEP3 108.40 ± 14.03%, Tg PEP3 99.96 ± 13.05, p>0.05). The data are shown as a percentage of the values of WT mice treated with inPEP3.

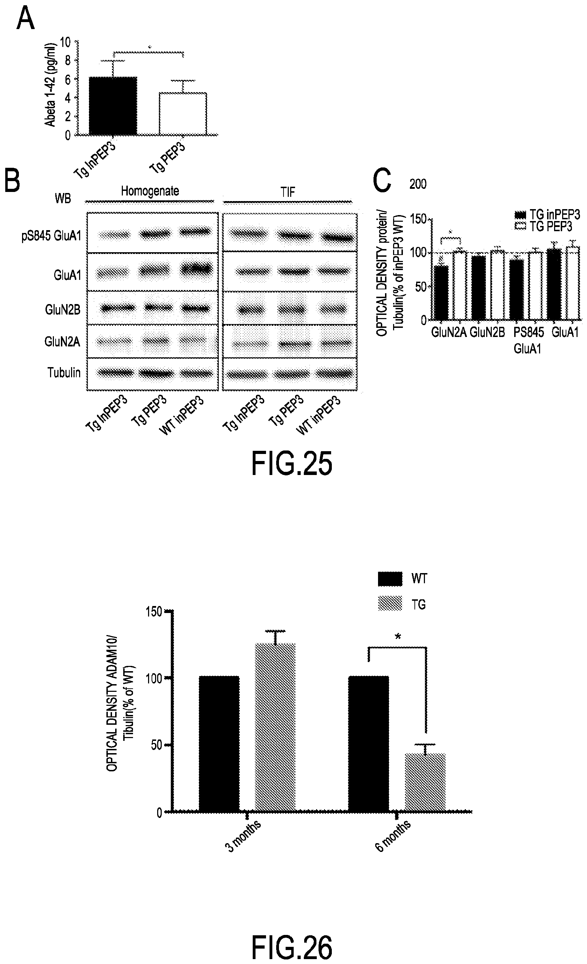

Figure 25 shows the results of the treatment of APP/PS1 mice of 9 months for 14 days with the active peptide PEP3 (SEQ ID NO:1) or the inactive peptide inPEP3 (SEQ ID NO:5) which reduces the levels of Aβ and modifies the composition of the glutamate receptors in synapsis. A. ELISA test of the levels of Aβ in APP/PS 1 mice treated for 14 days with PEP3 (TgPEP3) or with PEP3-inactive (Tg inPEP3). Significant reduction in the levels of Aβ in APP/PS1 mice treated with PEP3 and compared with APP/PS 1 mice treated with inPEP3 (Tg inPEP3 6.15 ± 0.63 pg/ml, Tg PEP3 4.47 ± 0.45 pg/ml, *p<0.05). B and C Data related to the levels of subunits of the glutamate receptors in the total homogenate and in the synaptic fraction (TIF) obtained from WT mice treated for 14 days with inPEP3 and in APP/PS1 mice treated for 14 days with PEP3 or with inPEP3. The GluN2A levels are reduced in Tg inPEP3 mice compared to WT inPEP3 mice (# p<0.05). Treatment with PEP3 significantly increases the synaptic levels ofGLUN2A in APP/PS 1 mice treated for 14 days with PEP3 with respect to mice treated with inPEP3 (Tg inPEP3 79.83 ± 4.42%, Tg PEP3 102.00 ± 5.10, *p<0.05). No significant changes emerged in relation to other synaptic proteins (GluN2B, GLUA1, pS845 GluA1). No modification to the total levels of proteins analysed in the different groups of animals was observed. The data are shown as a percentage of the values of WT mice treated with inPEP3.
Figure 26 illustrates the reduction in synaptic levels of ADAM10 in APP/PS1 mice at the age of 6 months but not 3 months. Hippocampi of WT animals and APP/PS 1 mice of 3 or 6 months were lysed and the synaptic fraction (TIF) was purified. The Western blot analysis of the TIF fraction shows a significant reduction in ADAM10 levels in APP/PS 1 mice of 6 months with respect to WT (APP/PS 1 34,50 ±3.80% percentage of WT, *p<0.05).

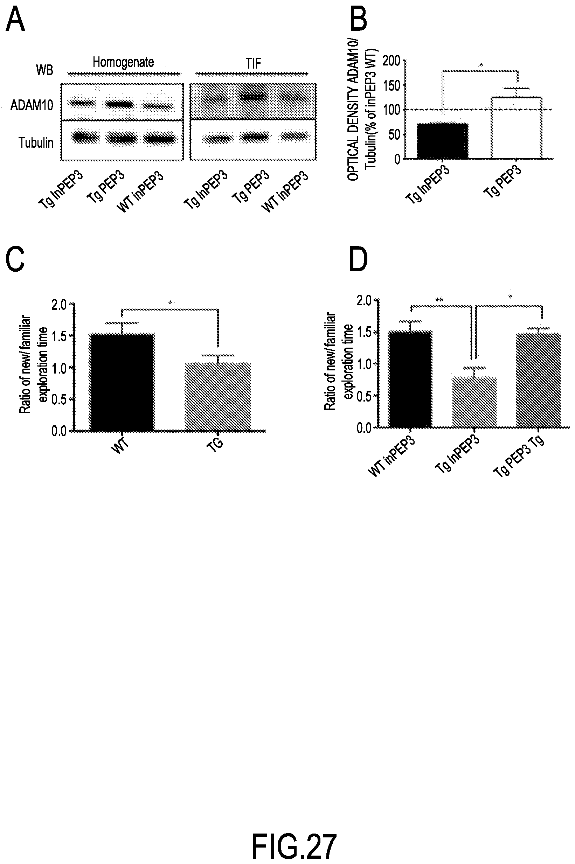

Figure 27 shows the results of the treatment of APP/PS1 mice of 6 months for 14 days with the active peptide PEP3 (SEQ ID NO:1) or the inactive peptide inPEP3 (SEQ ID NO:3) which restores the ADAM10 levels in synapsis and improves cognitive performance. Panels A and B. Data related to the levels of ADAM10 obtained through Western Blotting analysis of samples of homogenate and TIF obtained from WT and APP/PS 1 mice treated for 14 days with inPEP3 or with PEP3. Significant increase in the synaptic levels of ADAM10 in APP/PS1 mice treated with PEP3-active and compared with APP/PS 1 mice treated with inPEP3 (Tg inPEP3 69.94 ± 3.30%, Tg PEP3 125.00 ± 18.53, *p<0.05). No modification to the total levels of ADAM10 in the different groups of animals was observed. The data are shown as a percentage of the values of WT mice treated with inPEP3. Panel C. Data obtained through Y-maze test in untreated WT mice (1.53 ± 0.17) and APP/PS1 (1.06 ± 0.13). The significant reduction (* p<0.05) of the ratio of the new-arm/familiar-arm exploration time clearly shows the presence of a cognitive dysfunction in transgenic mice with respect to WT, as APP/PS1 mice explore the new arm less. Panel D. Data obtained through Y-maze test in WT mice treated for 14 days with inPEP3 (1.51 ± 0.16) and in APP/PS1 mice treated for 14 days with PEP3 (1.46 ± 0.09) or with inPEP3 (0.91 ± 0.20). The significant increase (*p<0.05) of the new arm/familiar arm exploration ratio in APP/PS 1 mice treated with PEP3-active shows an improvement in cognitive performance in this test compared with APP/PS1 mice treated with inPEP3.

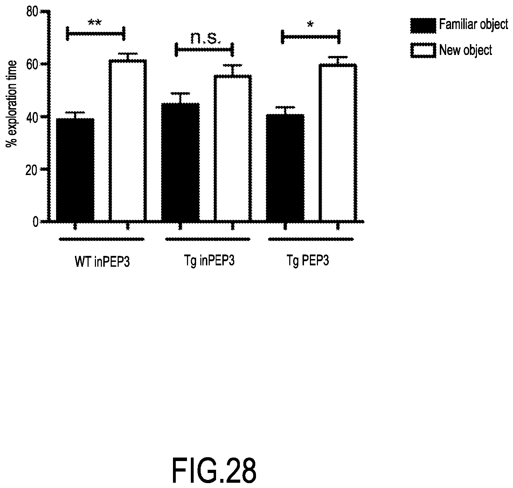

Figure 28 shows the results of NORT test performed on WT animals treated with inactive peptide inPEP3 (WT inPEP3) and APP/PS1 mice of 6 months treated with active peptide PEP3 (Tg PEP3) or inactive inPEP3 (Tg inPEP3). The graph shows the percentage of time spent by the different experimental groups exploring the familiar object and the new object.

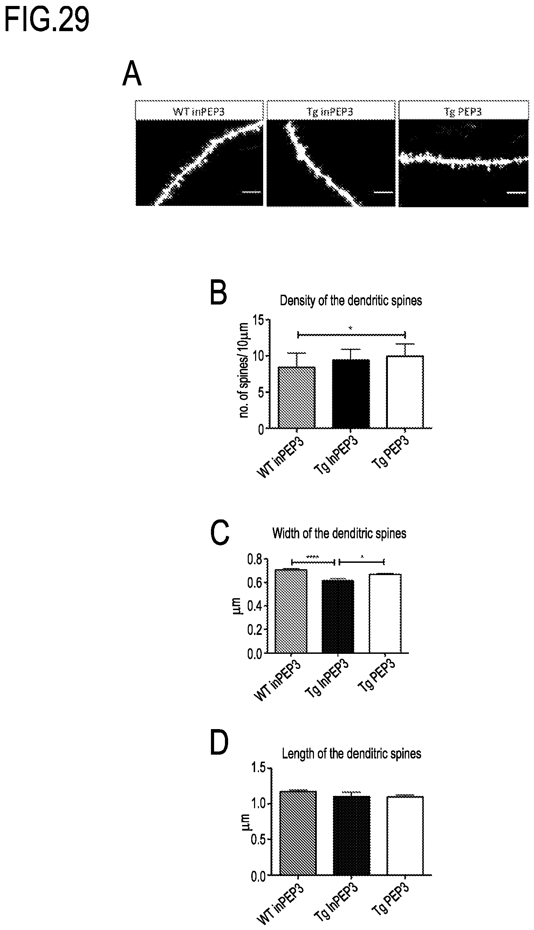

Figure 29 shows thea nalysis of the morphology and density of the dendritic spines following 14 days' treatment of WT animals with inactive peptide inPEP3 (WT inPEP3) and APP/PS1 mice of 6 months with active peptide PEP3 (Tg PEP3) or inactive inPEP3 (Tg inPEP3).

For the purpose of better illustrating the invention, the following examples are now provided, which are to be considered illustrative and non-limiting examples thereof.

### EXAMPLE 1: Design of the peptides

### MATERIALS AND METHODS

To better characterize the interaction between ADAM10 and AP2 the following bioinformatics and computational biophysics type analyses were performed: multiple sequence alignment, pattern recognition proteins, secondary structure prediction, modelling by homology, molecular dynamics, molecular docking.

### RESULTS

As demonstrated in the work published in 2013 by Marcello E. et al. [23], the ADAM10 link domain for the protein complex AP2 is comprised of two positively loaded amino acid residues, R⁷³⁵ and R⁷³⁷, and a hydrophilic amino acid Q⁷³⁶ (domain ⁷³⁵RQR⁷³⁷). In the same work it was demonstrated that both residues R play a key role in the interaction between ADAM10/AP2, as the double mutation of these residues in A leads to the complete loss of the link. To better characterize this interaction, different analyses were performed, both of the bioinformatics and computational biophysics type.

The information coming from the bioinformatics analyses enabled the amino acids to be identified directly and therefore physically involved in the interaction between ADAM10 and the complex AP2. In particular, the residues P⁷³³, P⁷³⁴, Q⁷³⁶ and R⁷³⁷ present on the cytoplasmic tail of ADAM10 interact with the residues F⁴⁶⁹, G⁴⁷² and E⁴⁷¹ of the subunit β2 of AP2. The residue D⁴³⁷ present on the subunit β2 of the complex AP2 is positioned so as to establish an electrostatic interaction with the residue R⁷³⁵ of the cytoplasmic tail of ADAM10. These results have also made it possible to predict the most probable mode of interaction between the two macromolecules ADAM10 and subunits β2 of AP2.

In light of these results, and with the objective of increasing the synaptic localization of ADAM10, with the consequent increase in the enzyme activity thereof, we decided to develop an instrument that could interfere with the endocytic mechanism of ADAM10. For this purpose, 3 active cell permeable peptides (CPP) were generated (SEQ ID NO:1, SEQ ID NO:2, SEQ ID NO:3) able to interfere in the formation of the bond between ADAM10/AP2.

The CPPs are comprised of the sequence of the TAT protein of the HIV-1 virus (TAT - YGRKKRRQRRR), which offers the ability to cross the biological membranes, fused with the sequence of the domain of the C-terminal tail of ADAM10 responsible for the interaction with the complex AP2.

The design of the 3 peptides was performed exploiting the computer-aided peptide design approach. In particular, the design was performed considering the sequence of the C-terminal tail of ADAM10 and that the function of these peptides was to inhibit a protein-protein interaction (i.e. ADAM10/AP2). In general, secondary structure elements (SSEs) play a key role in protein-protein interactions and it has been demonstrated that α-helices are the most common SSEs identified on the surface of protein-protein interactions For this reason, the sequences of peptides were modified to form SSEs that are stable in solution. Finally, to prevent the formation of non-functional interactions between peptide residues and positive amino acids of the carrier (i.e. Tat), a short spacer sequence was added between TAT and the active peptide.

The sequences of the three active peptides are:
- TAT-GGSG-QPPRQRPRG (SEQ ID NO:1) corresponding to PEP3
- TAT-GGSG-LPPRQRPRL (SEQ ID NO:2) corresponding to PEP2
- TAT-GGSG-QPPRQRPRE (SEQ ID NO:3) corresponding to PEP 1

The spacer sequence was represented by the GGSG amino acids whereas, as previously explained, the triplet RQR (indicated in formula I as X₂X₃X₄) represents the fulcrum region for inhibitory activity.

On the basis of these considerations, peptides with the following variations of the RQR triplet can be considered equivalent to the original peptides: KQK, RQK, KQR, RNR, KNR, RNK.

4 corresponding inactive peptides (inPEP1, inPEP2, inPEP3) were also developed, where the amino acid R of the sequence RQR is substituted with E, used as a control in every experiment having the following amino acid sequences:
- inPEP3: YGRKKRRQRRRGGSGQPPEQEPRG (SEQ ID NO:5)
- inPEP2: YGRKKRRQRRRGGSGLPPEQEPRL (SEQ ID NO:6)
- inPEP1: YGRKKRRQRRRGGSGQPPEQEPRE (SEQ ID NO:7).

### EXAMPLE 2: Validation of peptides in vitro

### MATERIALS AND METHODS

The acute hippocampal slices were obtained from rat brain. After carefully removing the meninges, the hippocampus was isolated and dissected using the McIlwain tissue chopper. The hippocampal slices were then transferred into supports containing Krebs buffer, in the presence of a constant flow of 95% O2 and 5% CO2 5% (v/v). The acute slices were kept for 45 min at ambient temperature before proceeding with treatment with the peptide.

For the treatments of the acute hippocampal slices, all the peptides were diluted in Krebs buffer at a concentration of 10µM. The treatments were performed for 30 minutes and subsequently the sample was immediately frozen.

The primary cultures were prepared from hippocampi of rat embryos as described in [20]. The cultures were grown in media containing Neurobasal (Life Technologies), B27 2% (Life Technologies), GlutaMAX 1% (Life Technologies) and Penicillin/Streptomycin 1% (Pen/Strep; Life Technologies), a 37 °C with 95% humidity and 5% CO2 for 14 days. The cells were treated by diluting the peptides in the complete growth medium, at a final concentration of 1 µM. The treatment was performed for 30 minutes at 37°C, then the neurons were lysed.

For the co-immunoprecipitation experiments, aliquots of 40-100 µg of lysate were incubated at 4°C in RIA 1X buffer (NaCl 200 mM, EDTA 10 mM, Na₂HPO₄ 10 mM, Nonidet P-40, 0.5% and 0.1% SDS) in a final volume of 150 µl with 1-2 µg of antibody. The following day the protein complex A/G (Santa Cruz Biotechnology) was added, conjugated with agarose marbles, and the incubation was continued for 2 hours. After three washes with RIA buffer the samples were loaded onto SDS-PAGE and analysed through Western Blot.

The fluorescence-based antibody uptake in COS-7 cells was performed as in [23].

The cross-link test with Bis(sulfosuccinimidyl)suberate (BS³) was performed on acute hippocampal slices as described in [23].

The Triton insoluble fraction (TIF) was purified as described in [20,23], the proteins separated through SDS-PAGE and analysed through Western Blot.

The quantization of the Western Blot analyses was performed through an image analysis program (ImageLab -BioRad). The levels of proteins were expressed as optical density (OD) related to the different measurements and were normalized on respective tubulin levels.

For the statistical analysis of the data the software GraphPad Prism 5 (GraphPad Software) was used. For the data that follow a normal distribution, the Student's T-test or the one-way ANOVA were applied followed the by Bonferroni post-hoc test to evaluate a statistically significant difference between samples treated with inactive peptides inPEP and with active peptides PEP. The data are expressed as mean ± SEM.

### RESULTS

As three different CPP peptides were developed, the first step of the study focused on testing the efficacy thereof in interfering with the interaction between ADAM10 and the complex AP2.

Acute hippocampal slices from rats were treated both with active peptides and with the related inactive peptides, used at a concentration of 10 µM, for 30 minutes. The samples were then processed, and the total homogenate was used to evaluate the interaction levels between ADAM10 and the complex AP2. The co-immunoprecipitation experiment (co-IP), performed using an anti-ADAM10 antibody, made it possible to verify that two of the three peptides (PEP2 (SEQ ID NO:2) and PEP3 (SEQ ID NO:1)) were able to interfere with the interaction between ADAM10 and α-adaptin, one of the subunits that constitutes the complex AP2 (Figure 1A). In particular, the quantitative analyses (Figure 1B) show that PEP2 is able to reduce by 58.4% the interaction between ADAM10 and α-adaptin, while PEP3 causes a 62.6% reduction (ratio PEP2/inPEP2 vs CTRL p=0.0054; PEP3/inPEP3 vs CTRL p=0.003).

Subsequently we confirmed these results in another biological model, focusing only on the peptides PEP2 and PEP3. Primary cultures of hippocampal neurons of rats were treated at day *in vitro* (DIV) 14 with both active CPPs and the corresponding inactive peptides, at the concentration of 1 µM. After 30 minutes, the cells were processed and the cell lysate was used for performing the co-IP analyses. As shown in Figure 1C, the treatment with both the active CPPs reduces the interaction between ADAM10 and two subunits of the complex AP2, specifically α-adaptin and µ2. The related quantitative analyses (Figure 1D), show that treatment with PEP2 and PEP3 determines a reduction in the levels of interaction between ADAM10 and the complex AP2, respectively of 66% and 89% with regard to the subunit α-adaptin (ratio PEP2/inPEP2 vs CTRL p=0.0019; PEP3/inPEP3 vs CTRL p=0.0013), and of 48% and 78% for the subunit µ2 (ratio PEP2/inPEP2 vs CTRL p=0.002; PEP3/inPEP3 vs CTRL p=0.0001).

As the endocytosis process mediated by clathrin is a common endocytosis mechanism of the proteins of the plasma membrane, we investigated whether the use of CPPs could alter the bond of AP2 with other endocytosis partners. For that purpose the interaction levels between the complex AP2 and the protein APP [28], the subunit GluA1 of the receptor AMPA [29] and the subunit β3 of the receptor GABA-A [30] were evaluated. In particular, the β3 subunit of the GABA-A receptor has a binding site for AP2 containing three residues R (405 RRR 407), making such sequence highly homologous to the "RQR" pattern present in the C-terminal tail of ADAM10 [23]. The samples obtained from the acute hippocampal slices treated both with PEP2 and with PEP3 (10 µM) or with the related controls, were co-immunoprecipitated using an anti-APP C-terminal antibody to detect the protein APP, an anti-GluA1 and anti-GABA-A-β3 antibody. The Western Blot analyses, performed by detecting the protein α-adaptin, do not display any changes in the interaction levels between the complex AP2 and the other target proteins, in the samples treated with both peptides (Figure 2). These results demonstrate that the CPPs specifically interfere with the interaction between ADAM10 and the complex AP2.

As the complex AP2 is responsible for the internalization of ADAM10, to study the functional meaning of the activity of the CPPs, we performed different tests to determine the efficacy of CPPs in interfering with the endocytosis of ADAM10 and in determining an increase in the localization of ADAM10 at membrane and synapsis level.

Firstly we performed a fluorescence-based antibody uptake test to evaluate the internalization of ADAM10 in COS7 cells. We transfected TacADAM10-RAR, a chimera of the surface reporter protein Tac (subunit α of the human receptor of IL-2) [31], with a modified version of the C-terminal tail of ADAM10, which presents a mutation in the retention sequence in the endoplasmic reticulum (ER) [32]. This mutation allows the release of ADAM10 from the ER and its localization in membrane [32]. The COS7 cells transfected with TacADAM10-RAR were treated with PEP2 or PEP3 and with the respective control peptides at the concentration of 1µM for 30 minutes. The TacADAM10-RAR chimeras were first marked through incubation with the anti-Tac antibody in live cells at 4°C, so as to block the trafficking at the membrane. Subsequently, the cells were brought back to 37°C to restore the endocytosis mechanism, or kept at 4°C as a control. The accumulation in the intracellular compartment, of the TacADAM10-RAR chimeras bonded to the antibody and the remaining quantity thereof on the surface were monitored using quantitative immunofluorescence staining in permeabilization and non-permeabilization conditions. The ratio between the intracellular fluorescence and that of the membrane enabled the internalization index to be evaluated, which was used as a control of the variable expression of TacADAM10-RAR chimeras in the different cells.

We discovered that the internalization index of the cells treated with PEP3 was significantly lower with respect to those treated with inPEP3 (Figure 3, panels A and B; internalization index, TacADAM10-RAR PEP3 = -12.85 ± 1.61%, TacADAM10-RAR inPEP3 = +1.30 ± 2.82% *p=0.0489). However, treatment with PEP2 did not significantly modify the internalization index of TacADAM10-RAR (Figure 3, panels A and B; internalization index, TacADAM10-RAR PEP2 =-10.95 ± 15.82% p>0.05, TacADAM10-RAR inPEP2 = -1.84 ± 13.92% p>0.05). This test demonstrates the ability of PEP3 to interfere with the internalization process of ADAM10, whereas the data obtained related to PEP2 show a reduction of the endocytosis of ADAM10, although not significant.

To better study the effect of both CPPs on the ADAM10 levels in membrane, acute hippocampal slices of rat were treated with PEP2 or PEP3 (or the respective control peptides) at the concentration of 10µM for 30 minutes, and the cross-link test with Bis(sulfosuccinimidyl)suberate (BS³) was performed. The membrane is impermeable to BS³, and it is able to covalently bond the proteins expressed on the cell surface. Treatment with BS³ produces aggregates of membrane proteins characterized by a high molecular weight, which have trouble entering the gel. As the intracellular proteins, instead, are not modified by such treatment, the quantity of intracellular proteins can be determined through Western Blot and reflects the levels of proteins inserted in membrane [23]. After treatment with CPPs in the presence or absence of BS³, the hippocampal rat slices were processed and the intracellular pool of ADAM10 was evaluated. As shown in the immunoblot images shown in Figures 4A and 4B, there is a substantial reduction of the intracellular levels of ADAM10 in the event that the slices were exposed to PEP2 or PEP3 in the presence of BS³. In particular, the quantitative analysis demonstrates that the intracellular levels of ADAM10 are significantly reduced following treatment with PEP2 and PEP3 with respect to control samples (figure 4 C-D, PEP2 74.22 ± 3.83% *p=0.009; inPEP2 102.90 ± 4.67% p>0.05; PEP3 37.52 ± 3.38% *p=0.0032; inPEP3 102.70 ± 9.73% p>0.05). As internal control for the experiment, we evaluated the levels of GluA1, the subunit of the receptor AMPA, which is enriched at synaptic membrane level. As shown in Figures 4A and 4B, in the presence of BS³, the subunit GluA1 of the receptor AMPA can just be detected in the intracellular pool, and the intracellular levels thereof are not modified by the treatment, hence demonstrating that the CPPs interfere specifically with the synaptic localization of ADAM10. These results demonstrate that both the CPPs not only interfere with the formation of the complex ADAM10/AP2 but also increase the surface expression of ADAM10.

After demonstrating that PEP2 and PEP3 can interfere with the interaction between the complex AP2 and ADAM10, hence reducing endocytosis, and as it has previously been demonstrated that ADAM10 is an integral protein of PSD [20], we decided to evaluate whether the active peptides could modulate the synaptic levels of ADAM10. For that purpose, primary hippocampal cultures were treated, at DIV14, with PEP2 or PEP3, and with the related inactive controls thereof, at the concentration of 1µM for 30 minutes. After treatment, the cells were lysed and processed to biochemically purify the triton insoluble fraction (TIF), which is enriched with proteins of the PSD. As shown in the immunoblot images provided in Figure 5A and in the respective quantitative analysis in figure 5B, treatment with both of the active peptides determines an increase of the synaptic localization of ADAM10 with respect to the control peptides (PEP2 +65.75 ± 3.19% **p<0.01 PEP2 vs inPEP2, PEP3 +114.90 ± 17.89% ***p<0.001, PEP3 vs inPEP3).

The set of results obtained from the experiments performed *in vitro* shows that the CPPs can effectively interfere with the formation of the complex ADAM10/AP2, and at the same time can increase the synaptic localization of the enzyme. In particular PEP3 seems to be more effective in interfering with the endocytosis of ADAM10 and in promoting the synaptic localization thereof.

### EXAMPLE 3: Validation of the efficacy of peptides according to the invention in vivo

### MATERIALS AND METHODS

For treatments *in vivo,* adult C57/BL6 mice were treated with the intraperitoneal injection of saline solution or peptides at a dose of 3 nmol/g (6 animals per experimental group). The animals were maintained in an enclosure at 25 ± 2 °C with an alternating light/dark cycle of 12 hours and had free access to water and food in the form of pellets (standard diet). 24 hours after injection the animals were sacrificed and the brain was rapidly dissected, divided into two parts and frozen at - 80 °C.

From one hemisphere the total homogenate used for the co-immunoprecipitation experiments was obtained and the TIF fraction was purified, whereas from the other hemisphere the soluble fraction was obtained for measuring sAPPα. The procedures are described in [20].

For the co-immunoprecipitation experiments, aliquots of 40-100 µg of lysate were incubated at 4°C in RIA 1X buffer (NaCl 200 mM, EDTA 10 mM, Na₂HPO₄ 10 mM, Nonidet P-40, 0.5% and 0.1% SDS) in a final volume of 150 µl with 1-2 µg of antibody. The following day the protein complex A/G (Santa Cruz Biotechnology) was added, conjugated with agarose marbles, and the incubation was continued for 2 hours. After three washes with RIA buffer the samples were loaded onto SDS-PAGE and analysed through Western Blot.

The proteins of TIF and of the soluble fraction were separated with SDS-PAGE and detected through Western Blot.

For the immunohistochemistry analyses, mice treated with the peptide were anaesthetized deeply and subjected to transcardial perfusion with 4% paraformaldehyde (prepared in PBS, pH 7.2-7.4), through the use of a peristaltic pump. The fixing liquid was inserted into the blood circulation through the left ventricle and after 10 minutes the brain was isolated. The sample was then post-fixed for 1 hour and 30 minutes at 4°C, then washed with PBS 1X and placed o/n in a 30% sucrose solution. The following day the sample was washed again with PBS 1X cut with the vibratome obtaining coronal sections of 50µm. Then the sections were stained through free-floating immunofluorescence technique. The fluorescence images were acquired using the Zeiss Confocal LSM510 Meta system with 63x objective at a resolution of 1024x1024 pixels.

The quantization of the Western Blot analyses was performed through an image analysis program (ImageLab -BioRad). The levels of proteins were expressed as optical density (OD) related to the different measurements and were normalized on respective tubulin levels.

For the statistical analysis of the data the software GraphPad Prism 5 (GraphPad Software) was used. For the data that follow a normal distribution, the Student's T-test or the one-way ANOVA were applied followed by the Bonferroni post-hoc test to evaluate a statistically significant difference between groups of animals treated with inactive peptides inPEP and with active peptides PEP. The data are expressed as mean ± SEM.

### RESULTS

Following the positive results obtained from the experiments performed *in vitro,* we decided to test the efficacy of the CPP peptides *in vivo.* In particular, the peptides SEQ ID NO:1 and SEQ ID NO:2 (PEP3 and PEP2, respectively) were tested.

The brain of mammals is protected by the blood-brain barrier (BBB), a highly selective barrier that separates the blood circulation from the extracellular brain fluid. The blood-brain barrier is a functional anatomic unit comprising endothelial cells connected to each other through the tight junction, which allows the passive diffusion of water, some gases and soluble lipid molecules, or the selective transport of glucose, amino acids or other substances necessary for the metabolic functions. As a first analysis we verified the ability of the CPPs to cross the BBB. For that purpose, C57BL/6 adult mice were treated with intraperitoneal injections of PEP2 or PEP3, or their respective controls, at the dose of 3 nmol/g. After 24 hours, the animals were anaesthetized and perfused with 4% paraformaldehyde (PFA). Subsequently, the brain was isolated and coronal sections of 50 µM were performed, in order to evaluate the presence of the peptide through immunohistochemistry tests.

The coronal sections were incubated with a specific antibody for the identification of the MAP2 neurons, and an antibody able to recognize the TAT sequence of HIV-1 present in the CPPs. As shown by the arrows in Figure 6, the presence of a specific signal of the anti-TAT antibody can be identified, which can only be detected in the coronal sections of brain of animals treated with CPPs. It is interesting to underline that the presence of the peptide is confined to neuron cell level with a prevalent perinuclear and dendritic distribution (Figure 6, panels B and C). In the brain of animals only treated with saline solution (Figure 6, panel A), such signal cannot be detected. These results demonstrate the ability of peptides to cross the blood-brain barrier, and therefore to reach the animal's brain.

After verifying the ability of the CPPs to cross the BBB, we evaluated the efficacy in interfering with the formation of the complex ADAM10/AP2. For that purpose C57BL/6 mice of six weeks were treated through intraperitoneal injection of PEP2 or PEP3 at the dose of 3 nmol/g and after 24 hours they were sacrificed. The brain was rapidly isolated and processed in order to obtain the total homogenate, the soluble fraction and the TIF. The samples of total homogenate were used to analyse the levels of formation of the complex ADAM10/AP2. The samples were immunoprecipitated using an anti-ADAM10 antibody and the interaction levels with the different subunits of the complex AP2 such as α-adaptin, β2-adaptin and µ2 were evaluated. In Figure 7A and in the related quantitative analysis shown in Figure 7B, it is shown that both peptides significantly interfere in the interaction between ADAM10 and the different subunits of AP2 (PEP2 α-adaptin -62.82 ± 12.78% **p< 0.01, β2-adaptin -73.36 ± 4.23% ***p<0.001, µ2 -54.39 ± 7.88% **p<0.01; PEP3 α-adaptin -64.96 ± 9.81% **p< 0.01, β2-adaptin -92.45 ± 4.02% ***p<0.001, µ2 -61.06 ± 11.63% **p<0.01).

These results demonstrate the ability of the CPPs to interfere with the formation of the complex ADAM10/AP2 *in vivo.*

To verify the efficacy of the CPPs *in vivo,* we analysed the synaptic localization and activity of ADAM10 towards the protein APP. To evaluate the synaptic levels of the enzyme we analysed the TIF fraction, whereas to evaluate the activity we used the soluble fraction, and in particular we measured the levels of sAPPα, which corresponds to the fragment released following the cutting of APP by ADAM10. The Western Blot images shown in Figure 8, panel A and C, demonstrate that the levels of ADAM10 present in the total homogenate are not influenced by treatment with active peptides (inPEP2 102.00 ± 5.24%, PEP2 98.48 ± 11.02% p>0.05, inPEP3 97.53 ± 10.85%, PEP3 100.00 ± 11.62% p>0.05); on the contrary we observed a large increase in the levels of ADAM10 in the synaptic fraction (Figure 8B). In particular, the quantitative analysis (Figure 8D) demonstrates that both of the active peptides increase the synaptic localization of ADAM10 by 50% with respect to the treatment performed with the respective inactive peptides (PEP2 151.70 ± 9.77% *p<0.05, PEP3 147.50 ± 7.10% *p<0.05). These results clearly demonstrate that the increase in the synaptic levels of ADAM10 are determined by a modification in its trafficking rather than an increase in its protein synthesis, as the total levels of the protein remain unvaried after treatment with the active peptide.

With regard to the activity of ADAM10, the analysis of the levels of sAPPα in the soluble fraction demonstrated that treatment with both the active peptides promotes the release thereof, suggesting a shift of the metabolism of APP towards the non-amyloidogenic route (Figure 9, inPEP2 130.80 ± 34.66%, PEP2 271.40 ± 36.90% *p<0.05, inPEP3 98.41 ± 9.69%, PEP3 155.50 ± 15.05% p<0.05).

These results demonstrate that the intraperitoneal injection of a single dose of 3 nmol/g of CPP can interfere with the formation of the complex ADAM10/AP2 and can determine an increase in the synaptic localization of ADAM10 and of its activity towards the protein APP.

### EXAMPLE 4: Analysis of the efficacy of different administration routes and different doses of the peptide PEP3 (SEQ ID NO:1)

### MATERIALS AND METHODS

To evaluate the best dose and administration route, C57/BL6 adult mice were treated with the intraperitoneal or subcutaneous injection of peptides at a dose of 3 nmol/g or 1 nmol/g (6 animals per experimental group). 24 or 48 hours after the injection the animals were sacrificed and the brain was rapidly dissected, divided into two hemispheres and frozen at -80 °C.

For the stability analyses of the effect, C57/BL6 adult mice were treated with intraperitoneal injection of peptides at a dose of 3 nmol/g and sacrificed 8 hours, 12 hours, 24 hours, 36 hours after treatment (6 animals per experimental group). From one hemisphere the total homogenate used for the co-immunoprecipitation experiments was obtained and the TIF fraction was purified, whereas from the other hemisphere the soluble fraction was obtained for measuring sAPPα. The procedures are described in [20].

For the co-immunoprecipitation experiments, aliquots of 40-100 µg of lysate were incubated at 4°C in RIA 1X buffer (NaCl 200 mM, EDTA 10 mM, Na₂HPO₄ 10 mM, Nonidet P-40, 0.5% and 0.1% SDS) in a final volume of 150 µl with 1-2 µg of antibody. The following day the protein complex A/G (Santa Cruz Biotechnology) was added, conjugated with agarose marbles, and the incubation was continued for 2 hours. After three washes with RIA buffer the samples were loaded onto SDS-PAGE and analysed through Western Blot.

The proteins of TIF and of the soluble fraction were separated with SDS-PAGE and detected through Western Blot.

The quantization of the Western Blot analyses was performed through an image analysis program (ImageLab -BioRad). The levels of proteins were expressed as optical density (OD) related to the different measurements and were normalized on respective tubulin levels.

For the statistical analysis of the data the software GraphPad Prism 5 (GraphPad Software) was used. For the data that follow a normal distribution, the Student's T-test or the one-way ANOVA were applied followed by the Bonferroni post-hoc test to evaluate a statistically significant difference between groups of animals treated with the inactive peptide inPEP3 or with the active peptide PEP3. The data are expressed as mean ± SEM.

### RESULTS

To evaluate the efficacy and toxicity of the treatment with CPPs, the peptide PEP3 (SEQ ID NO:1) was selected, as was shown to be the most effective in influencing the endocytosis of ADAM10 and in modifying the availability thereof at synaptic level.

To determine the experimental conditions to be used for a treatment lasting 14 days, we tested two doses (1 nmol/g or 3 nmol/g), different administration routes, i.e. sub-cutaneous or intraperitoneal injection, and we checked the stability of the effect induced by PEP3 24 hours and 48 hours after administration.

C57BL/6 adult mice were subjected to intraperitoneal or sub-cutaneous injection of PEP3 or inPEP3 at the doses of 3 nmol/g or 1 nmol/g. The animals were sacrificed after 24 hours or 48 hours, their brains were collected and from these the soluble protein fraction and the TIF were purified. For each experimental group, the interaction between ADAM10 and the complex AP2 were evaluated, and the availability and activity of ADAM10 at synaptic level were evaluated.

The co-immunoprecipitation experiments performed in the total homogenate obtained from the brain of animals treated through intraperitoneal injection (Figure 10, panel A), showed that PEP3 can only reduce the ADAM10/AP2 interaction at the higher dose (3 nmol/g) and that its effect only lasts for 24 hours, as at 48 hours no modifications of the levels of the complex ADAM10/AP2 were observed. In fact, the quantitative analysis shown in Figure 10, panel B only shows a reduction of the levels of ADAM10/AP2 in the brain of animals treated with the dose of 3 nmol/g and sacrificed 24 hours after administration (PEP3/inPEP3 -35.74 ± 7.80% p<0.05).

In animals treated through intraperitoneal injection, we subsequently evaluated the levels of ADAM10 in the synaptic fraction TIF.

The immunoblot image shown in Figure 10, panel C and the respective quantitative analysis in Figure 10, panel D, show that only the dose of 3 nmol/g of PEP3 determines a significant increase in ADAM10 at synaptic level 24 hours after administration, but not 48 hours after (PEP3 113.70 ± 2.83% p<0.05). PEP3 is not shown to be effective at the lower dose (1 nmol/g). In line with these results, the immunoblot and the quantitative analysis of the levels of sAPPα performed in the soluble fraction of the brain (Figure 10, panels E, F) demonstrate that PEP3 positively modulates the activity of ADAM10 only in mice treated with the dose of 3 nmol/g and sacrificed after 24 hours (PEP3 151.60 ± 12.22% p<0.05). Neither the dose of 1 nmol/g, nor the time of 48 hours determine significant alterations to the release of sAPPα.

These results clarify that the peptide PEP3 (SEQ ID NO:1) is only effective if administered through intraperitoneal injection at the dose of 3 nmol/g and that its effect is only stable for 24 hours.

The next step is that of assessing a different administration method of the peptide, therefore the same analyses were repeated in mice treated by means of sub-cutaneous injection at the dose of 3 nmol/g or 1 nmol/g and sacrificed 24 hours or 48 hours after injection.

The co-immunoprecipitation experiments showed that PEP3 is not able to interfere with the ADAM10/AP2 interaction in any of the experimental groups (Figure 11, panels A and B). Furthermore, the sub-cutaneous injection of PEP3 does not alter the synaptic availability of ADAM10, as shown in the immunoblot performed in the TIF fraction (Figure 11, panels C and D). As envisaged, the activity of ADAM10 is not altered either. Both the immunoblot performed in the soluble brain fraction (Figure 11E), and the quantitative analysis (Figure 11F), demonstrate that there are no significant changes in the release of sAPPα when the animals treated with PEP3 are considered, with respect to those treated with inPEP3.

All these results demonstrate that sub-cutaneous injection cannot be considered a valid administration route, and that only intraperitoneal injection at the dose of 3 nmol/g represents the best condition to guarantee the efficacy of PEP3 in modulating the formation of the complex ADAM10/AP2 and therefore the activity of ADAM10.

We subsequently decided to evaluate the stability and efficacy of PEP3 at different times.

C57BL/6 adult mice were treated with PEP3 and inPEP3 through intraperitoneal injection at the dose of 3 nmol/g, they were sacrificed after 8, 12, 24 or 36 hours, and their brains were collected and the soluble protein fraction and the TIF were purified therefrom. We evaluated the ADAM10/AP2 interaction and the availability and synaptic activity of ADAM10. The samples of total homogenate, obtained from the brain of the animals, were immunoprecipitated with an antibody for ADAM10, and the co-IP with α-adaptin was used to evaluate the interaction between ADAM10 and the complex AP2. As shown in Figure 12A and confirmed by the quantitative analysis in Figure 12B, and as we expected, PEP3 reduces by 31% the interaction between ADAM10 and α-adaptin 24 hours after administration (PEP3/inPEP3 24 hours -31.00 ± 6.12% p<0.05). 36 hours after injection, we observed a 20% reduction, but which was not shown to be statistically significant (PEP3/inPEP3 36 hours -19.69 ± 16.86% p>0.05). As shown in Figure 12C and confirmed by the quantitative analysis in Figure 12D, PEP3 increases the synaptic availability of ADAM10 24 hours after administration (PEP3 24 hours 137.40 ± 6.32% p<0.05). Finally, 12 and 36 hours after injection, we only observed a slight, but not statistically significant, increase in the synaptic levels of ADAM10. Therefore, the activity of ADAM10 shows the same trend. The soluble cerebral fraction was used to determine the levels of sAPPα and PEP3 increases by about 30% the levels of sAPPα 24 hours after administration (PEP3, 24 ore 126.30 ± 6.23% p<0.05). 12 and 36 hours after administration, it is possible to observe only a slight increase in sAPPα levels, which is not however statistically significant (PEP3, 12 ore 112.70 ± 5.62% p>0.05; PEP3 36 ore 120.00 ± 5.70%).

### EXAMPLE 5: Analysis of efficacy and toxicity of 14 days' administration of the peptide PEP3 in vivo in wild-type mice

### MATERIALS AND METHODS

C57/BL6 adult mice were treated for 14 days with daily intraperitoneal injection of saline solution or peptides at a dose of 3 nmol/g (6-10 animals per experimental group). The animals were maintained in an enclosure at 25 ± 2 °C with an alternating light/dark cycle of 12 hours and had free access to water and food in the form of pellets (standard diet).

After 14 days of treatment the animals were subjected to the Novel Object Recognition Test (NORT). Such test is structured in three phases: habituation, familiarization and testing. In the first phase, the animal is placed in an empty arena and left free to explore the environment for 5 minutes. The day after, during the familiarization phase, the animal is placed in the same arena in the presence of two objects, identical in shape and size, for 10 minutes. Then the time spent by the animal exploring the individual object is recorded. On the third day, the animal is placed in the area in the presence of an identical object to that used in the familiarization phase and a new one. In this phase the difference in time spent by the animal exploring the familiar object and the new object is evaluated. The test shows the natural preference of the animal towards the exploration of the new object with respect to the one that is considered familiar. In both phases of the test, the objects are positioned symmetrically and opposite inside the arena, and all the sessions are recorded with the aid of a video camera.

After the NORT the animals were sacrificed and the brain was rapidly dissected and divided into two hemispheres: one was frozen at -80°C and the other one fixed in formalin and included in paraffin.

The blood, collected through cardiac puncture, was analysed with a laser counter (ADVIA 120, Siemens) using specific software for the murine species. From each sample a blood smear was prepared, stained with the May Grünwald-Giemsa method, and used for the morphological analysis of the leukocytes, the red blood cells and the platelet estimate. After the automatic counting of the cells and the preparation of the smear, all the samples were centrifuged and the plasma was transferred into separate test tubes. The analysis of the plasma samples was performed through automatic spectrophotometer (ILAB-300, Instrumentation Laboratory). All the results were compared with the values provided by the company Charles River for mice of the same strain and age.

After the sacrifice of the treated animal, a portion of liver and a brain hemisphere were fixed in 10% formalin for 48 hours, and included in paraffin. From each block of paraffin, serial sections 5/6µm thick were obtained. The sections were then stained with haematoxylin-eosin or processed through immunohistochemistry.

For the dendritic spine analysis, the protocol used is the one previously described by Kim et al [33], and envisages the use of DilDye lipophilic tracer (Invitrogen) to allow the dendritic spines to be viewed under the confocal microscope. The analysis of the dimensions and morphology of the spines was performed with ImageJ software.

From one hemisphere the total homogenate was obtained used for the co-immunoprecipitation experiments and the TIF fraction was purified.

For the co-immunoprecipitation experiments, aliquots of 40-100 µg of lysate were incubated at 4°C in RIA 1X buffer (NaCl 200 mM, EDTA 10 mM, Na₂HPO₄ 10 mM, Nonidet P-40, 0.5% and 0.1% SDS) in a final volume of 150 µl with 1-2 µg of antibody. The following day the protein complex A/G (Santa Cruz Biotechnology) was added, conjugated with agarose marbles, and the incubation was continued for 2 hours. After three washes with RIA buffer the samples were loaded onto SDS-PAGE and analysed through Western Blot.

The proteins of TIF and of the soluble fraction were separated with SDS-PAGE and detected through Western Blot. The levels of sAPPα were measured using an ELISA kit (IBL international).

The quantization of the Western Blot analyses was performed through an image analysis program (ImageLab -BioRad). The levels of proteins were expressed as optical density (OD) related to the different measurements and were normalized on respective tubulin levels.

For the statistical analysis of the data the software GraphPad Prism 5 (GraphPad Software) was used. For the data that follow a normal distribution, the Student's T-test or the one-way ANOVA were applied followed by the Bonferroni post-hoc test to evaluate a statistically significant difference between groups of animals treated with the inactive peptide inPEP3 or with the active peptide PEP3. The data are expressed as mean ± SEM.

### RESULTS

In light of these results, a chronic 14-day treatment with the peptide PEP3 was performed (SEQ ID NO:1). For the entire duration of the treatment, on a daily basis, the weight and food intake of each animal were monitored and recorded, in order to evaluate any changes caused by the treatment. 24 hours after the last injection the mice were anaesthetized, and then the blood was taken through cardiac puncture, whereas the brain and liver of the animal were rapidly dissected to perform biochemical or immunohistological analyses.

### Effects on body weight and on food intake following 14 days' treatment

The body weight and food intake were monitored and recorded on a daily basis. As shown in panel A (Figure 13) treatment with the active or inactive peptide does not affect the weight increase; further, the food intake detected for animals treated with the peptides (active or inactive) is comparable to that of animals treated with saline (Figure 13B).

These results indicate that treatment with CPP does not interfere with the animal's normal metabolism.

### Haematological and histological analyses

### Blood count and platelet count

The blood count analyses performed on the three experimental groups (mice treated with saline, mice treated with PEP3 (SEQ ID NO:1) and mice treated with the control inPEP3 (SEQ ID NO:5), showed that most of the mean and median values fall within the reference ranges, with the exception of the MCV (mean corpuscular volume), the value of which, for some animals, is above the reference range. On the contrary, the MCH (mean corpuscular haemoglobin) and MCHC (mean corpuscular haemoglobin concentration) values were lower than the reference values for all the mice analysed, suggesting that this difference may depend on analytical or pre-analytical factors compared to the instruments/methods (not known) used to define the reference values (Table 1). The following Table 1 shows the results related to the blood count and the platelet count. The range values are provided by Charles River for C57BL mice aged between 56-70 days, whose blood was collected by cardiac puncture after euthanasia.

**Table 1**

| **SAMPLE** | **Red blood cells x10⁶/µL** | **Hb (g/dL)** | **(Ht) (%)** | **Vol. Mean Corp. Vol. (MCV) (fL)** | **Mean Corpuscula r Hb (MCH) (Pg)** | **Mean Corpuscula r Hb Conc. MCHC (%)** | **Pit x10³/µL** | **Red cell distribution width (RDW) (%)** | **Estimate Plt/ Aggregate s** |
|---|---|---|---|---|---|---|---|---|---|
| SALINE | 8.84 | 12.1 | 46.6 | 52.7 | 13.7 | 26 | 1199 | 19.6 | A/+++ |
| | 8.67 | 11.7 | 45.7 | 52.7 | 13.5 | 25.6 | 382 | 19.6 | A/+++ |
| | 9 | 12 | 45.9 | 51 | 13.3 | 26.1 | 345 | 19.6 | A/+++ |
| | 8.58 | 11.4 | 42.7 | 49.8 | 13.3 | 26.7 | 1327 | 21 | A/+++ |
| | 9.11 | 12.1 | 46.7 | 51.3 | 13.3 | 25.9 | 737 | 21.2 | A/+++ |
| | 8.93 | 12.1 | 47.8 | 53.5 | 13.5 | 25.3 | 657 | 20.1 | A/+++ |
| **Mean ± SD (Median)** | **8.86** | **11.9** | **45.9** | **51.8** | **13.4** | **25.9** | **775** | **20.2** | |
| | **± 0.20** | **± 0.3** | **± 1.7** | **± 1.4** | **± 0.2** | **± 0.5** | **± 410** | **± 0.7** | |
| | **-8.89** | **-12.1** | **-46.3** | **-52** | **-13.4** | **-26** | **-697** | **-19.9** | |
| inPEP3 | 9.36 | 12.6 | 50.1 | 53.5 | 13.5 | 25.1 | 522 | 20.4 | A/+++ |
| | 9.34 | 12.6 | 49.5 | 53 | 13.5 | 25.5 | 661 | 20.6 | A/+++ |
| | 9.01 | 12 | 47.8 | 53.1 | 13.3 | 25.1 | 656 | 21.4 | A/+++ |
| | 9.21 | 12.4 | 48.7 | 52.9 | 13.5 | 25.5 | 341 | 21.8 | A/+++ |
| | 9.04 | 12.1 | 48 | 53.1 | 13.4 | 25.2 | 998 | 19.6 | A/+++ |
| | 8.97 | 12 | 47.3 | 52.7 | 13.4 | 25.4 | 790 | 20.1 | A/+++ |
| **Mean ± SD (Median)** | **9.16** | **12.3** | **48.6** | **53.1** | **13.4** | **25.3** | **661** | **20.7** | |
| | **± 0.17** | **± 0.3** | **± 1.1** | **± 0.3** | **± 0.1** | **± 0.2** | **± 224** | **± 0.8** | |
| | **-9.13** | **-12.3** | **-48.4** | **-53.1** | **-13.5** | **-25.3** | **-659** | **-20.5** | |
| PEP3 | 8.14 | 10.9 | 41.5 | 51 | 13.4 | 26.3 | 861 | 18.7 | A/+++ |
| | 8.75 | 11.7 | 47.4 | 54.2 | 13.4 | 24.7 | 711 | 19.3 | A/+++ |
| | 9.04 | 12.5 | 48.5 | 53.7 | 13.8 | 25.8 | 1333 | 19.9 | A/+++ |
| | 8.94 | 11.9 | 48 | 53.7 | 13.3 | 24.8 | 391 | 19.9 | A/+++ |
| | 8.84 | 11.9 | 46.3 | 52.4 | 13.5 | 25.7 | 1302 | 19.7 | A/+++ |
| | 8.72 | 11.9 | 45.7 | 52.4 | 13.6 | 26 | 1394 | 19.7 | A/+++ |
| **Mean ± SD (Median)** | **8.74** | **11.8** | **46.2** | **52.9** | **13.5** | **25.6** | **999** | **19.5** | |
| | **± 0.32** | **± 0.5** | **± 2.5** | **± 1.2** | **± 0.2** | **± 0.7** | **± 408** | **± 0.5** | |
| | **-8.8** | **-11.9** | **-46.9** | **-53.1** | **-13.5** | **-25.8** | **-1082** | **-19.7** | |
| Reference range | 9.51±1.2 5 | 14.3± 2.6 | 47.0± 7.3 | 49.4±3.9 | 14.9±1.1 | 30.3±3.0 | N/A | 1350±338 | |

| | | | | | | | | | |
|---|---|---|---|---|---|---|---|---|---|
| A= appropriate platelet estimate; +, ++, +++ considering the presence of platelet aggregates detected in the blood smear that make the analysis difficult; Hb= haemoglobin; Ht= haematocrit; Plt= platelet. | | | | | | | | | |

The statistical analysis (Figure 14) does not show any significant differences in Ht (haematocrit), Hb (haemoglobin), MCV, MCH, MCHC values, but shows a statistically higher number of RBCs (red blood cells) in mice treated with the inactive peptide with respect to the other experimental groups. This can be explained by the moderate dehydration of the sample, however this does not represent a significant biological datum as all the values remain within the range of reference values.

Although the platelet count is very low in all the animals considered, the morphological analysis of the blood smears highlighted that the platelets were always present in an appropriate number, but often in the form of aggregates. This suggests that the low platelet count obtained may be caused by an analytical artefact (as the platelet aggregates cannot pass into the counting chamber of the analyzer), as none of the treated mice is affected by thrombocytopenia.

### Leukogram

Except for the basophils and eosinophils, the total count and white blood cell (WBC) differential is lower with respect to the range values in all the animals treated. However, we noticed that the reference range values provided by the company Charles River are particularly high with respect to other values provided for other control groups and in other scientific articles in which the same animal strain is analysed [34,35]. Justification for these differences found is provided by the pre-analytical factors used by Charles River in determining the different reference ranges (such as the method used, the type of sampling used, etc.). Despite this, the analyses of the data performed both individually and by examining the mean and median values in the different groups, show some differences in the values referring to mice treated with the active peptide with respect to the control mice (Table 2).

The following Table 2 illustrates the results of the white blood cell count. The reference values are provided by Charles River for C57BL mice of 56-70 days.

**Table 2**

| **Sample** | **White blood cells x10³/µL** | **Neutrophils x10³/µL** | **Lymphocytes x10³/µL** | **Monocytes x10³/µL** | **Eosinophils x10³/µL** | **Basophils x10³/µL** | **Estimate Plt /Aggregates** |
|---|---|---|---|---|---|---|---|
| SALINE | 2.2 | 0.73 | 1.3 | 0.11 | 0.04 | 0.02 | A/+++ |
| | 2.11 | 0.44 | 1.54 | 0.08 | 0.04 | 0 | A/+++ |
| | 3.9 | 0.47 | 3.24 | 0.12 | 0.08 | 0 | A/+++ |
| | 4.07 | 1.1 | 2.69 | 0.2 | 0.08 | 0 | A/+++ |
| | 2.47 | 0.25 | 2.07 | 0.1 | 0.02 | 0.02 | A/+++ |
| | 1.76 | 0.16 | 1.48 | 0.07 | 0.04 | 0.02 | A/+++ |
| **Mean ± SD (Median)** | **2.7517** | **0.52** | **2.05** | **0.11** | **0.05** | **0.01** | |
| | **± 0.9834** | **± 0.34** | **± 0.77** | **± 0.05** | **± 0.02** | **± 0.01** | |
| | **(-2.335).** | **(-0.46).** | **(-1.81).** | **(-0.1).** | **(-0.04).** | **(-0.01).** | |
| inPEP3 | 2.61 | 0.261 | 2.2185 | 0.1044 | 0.0261 | 0 | A/+++ |
| | 1.55 | 0.2325 | 1.271 | 0.0155 | 0.031 | 0 | A/+++ |
| | 4.48 | 2.2848 | 1.9712 | 0.1792 | 0.0448 | 0 | A/+++ |
| | 6.2 | 2.79 | 2.976 | 0.372 | 0.062 | 0 | A/+++ |
| | 1.8 | 0.216 | 1.476 | 0.072 | 0.036 | 0 | A/+++ |
| | 1.98 | 0.1584 | 1.7028 | 0.0792 | 0.0396 | 0 | A/+++ |
| **Mean ± SD (Median)** | **3.1033** | **0.9905** | **1.9359** | **0.1371** | **0.0399** | **0** | |
| | **± 1.8494** | **± 1.2093** | **± 0.6117** | **±0.1268** | **±0.0126** | **± 0.0000** | |
| | **(-2.295).** | **(-0.2468).** | **(-1.837).** | **(-0.0918).** | **(-0.0378).** | **0** | |
| PEP3 | 6.83 | 0.68 | 5.74 | 0.27 | 0.14 | 0 | A/+++ |
| | 3.12 | 0.34 | 2.65 | 0.06 | 0.06 | 0 | A/+++ |
| | 4.55 | 0.46 | 3.82 | 0.18 | 0.09 | 0 | A/+++ |
| | 4.38 | 0.44 | 3.72 | 0.13 | 0.04 | 0.04 | A/+++ |
| | 5.87 | 0.65 | 4.81 | 0.29 | 0.12 | 0 | A/+++ |
| | 6.99 | 0.63 | 6.01 | 0.28 | 0.07 | 0 | A/+++ |
| **Mean ± SD (Median)** | **5.29** | **0.53** | **4.46** | **0.2** | **0.09** | **0.01** | |
| | **1.5286** | **± 0.14** | **± 1.29** | **± 0.09** | **± 0.04** | **± 0.02** | |
| | **(-5.21).** | **(-0.54).** | **(-4.32).** | **(-0.23).** | **(-0.08).** | **0** | |
| Reference range | 8.91±2.50 | 1.45±1.01 | 6.85±1.97 | 0.43±0.24 | 0.14±0.12 | 0.03±0.03 | |

It is interesting to highlight, as also shown by the statistical analysis in Figure 15, that the mice treated with the active peptide show a higher:
1) white blood cell (WBC) count with respect to the control;
2) lymphocyte count with respect to both controls, mice to which a saline solution was injected and mice treated with the inactive peptide, suggesting a possible chronic subcutaneous activation of the immune responses;
3) eosinophil count with respect to mice treated with the inactive peptide, indicating a possible allergic condition (although this datum is statistically significant, it does not represent a biologically significant datum, as all the values referring to animals treated with PEP3 fall within the reference range).

However, the morphological analysis performed on the blood smear did not show any pathological alterations within each experimental group.

### Clinical chemistry

We subsequently evaluated the liver and kidney functionality. The urea, creatinine and ALT (alanine aminotransferase) concentrations measured in the control and treated mice fell within the reference ranges (Table 3).

The following Table 3 shows the evaluation of the laboratory results. The reference values are provided by Charles River for C57BL mice of 56-70 days.

**Table 3**

| **SAMPLE** | **White blood cells x10³/µL** | **Neutrophils x10³/µL** | **Lymphocytes x10³/µL** | **Monocytes x10³/µL** | **Eosinophils x10³/µL** | **Basophils x10³/µL** | **Estimate Pit /Aggregates** |
|---|---|---|---|---|---|---|---|
| SALINE | 2.2 | 0.73 | 1.3 | 0.11 | 0.04 | 0.02 | A/+++ |
| | 2.11 | 0.44 | 1.54 | 0.08 | 0.04 | 0 | A/+++ |
| | 3.9 | 0.47 | 3.24 | 0.12 | 0.08 | 0 | A/+++ |
| | 4.07 | 1.1 | 2.69 | 0.2 | 0.08 | 0 | A/+++ |
| | 2.47 | 0.25 | 2.07 | 0.1 | 0.02 | 0.02 | A/+++ |
| | 1.76 | 0.16 | 1.48 | 0.07 | 0.04 | 0.02 | A/+++ |
| **Mean ± SD (Median)** | **2.7517** | **0.52** | **2.05** | **0.11** | **0.05** | **0.01** | |
| | **± 0.9834** | **± 0.34** | **± 0.77** | **± 0.05** | **± 0.02** | **± 0.01** | |
| | **(-2.335).** | **(-0.46).** | **(-1.81).** | **(-0.1).** | **(-0.04).** | **(-0.01).** | |
| inPEP3 | 2.61 | 0.261 | 2.2185 | 0.1044 | 0.0261 | 0 | A/+++ |
| | 1.55 | 0.2325 | 1.271 | 0.0155 | 0.031 | 0 | A/+++ |
| | 4.48 | 2.2848 | 1.9712 | 0.1792 | 0.0448 | 0 | A/+++ |
| | 6.2 | 2.79 | 2.976 | 0.372 | 0.062 | 0 | A/+++ |
| | 1.8 | 0.216 | 1.476 | 0.072 | 0.036 | 0 | A/+++ |
| | 1.98 | 0.1584 | 1.7028 | 0.0792 | 0.0396 | 0 | A/+++ |
| **Mean ± SD (Median)** | **3.1033** | **0.9905** | **1.9359** | **0.1371** | **0.0399** | **0** | |
| | **± 1.8494** | **± 1.2093** | **± 0.6117** | **±0.1268** | **±0.0126** | **± 0.0000** | |
| | **(-2.295).** | **(-0.2468).** | **(-1.837).** | **(-0.0918).** | **(-0.0378).** | **0** | |
| PEP3 | 6.83 | 0.68 | 5.74 | 0.27 | 0.14 | 0 | A/+++ |
| | 3.12 | 0.34 | 2.65 | 0.06 | 0.06 | 0 | A/+++ |
| | 4.55 | 0.46 | 3.82 | 0.18 | 0.09 | 0 | A/+++ |
| | 4.38 | 0.44 | 3.72 | 0.13 | 0.04 | 0.04 | A/+++ |
| | 5.87 | 0.65 | 4.81 | 0.29 | 0.12 | 0 | A/+++ |
| | 6.99 | 0.63 | 6.01 | 0.28 | 0.07 | 0 | A/+++ |
| **Mean ± SD (Median)** | **5.29** | **0.53** | **4.46** | **0.2** | **0.09** | **0.01** | |
| | **1.5286** | **± 0.14** | **± 1.29** | **± 0.09** | **± 0.04** | **± 0.02** | |
| | **(-5.21).** | **(-0.54).** | **(-4.32).** | **(-0.23).** | **(-0.08).** | **0** | |
| Reference range | 8.91±2.50 | 1.45±1.01 | 6.85±1.97 | 0.43±0.24 | 0.14±0.12 | 0.03±0.03 | |

| | | | | | | | |
|---|---|---|---|---|---|---|---|
| ** expressed as urea in the blood | | | | | | | |

On the contrary, the values related to total proteins, total globulin and to a lesser extent, albumin, were lower with respect to the reference values in most of the control and treated animals. Furthermore, also the concentration of triglycerides was lower at the reference values both in the control mice and in the treated mice.

On the basis of a lack of clear signs of liver failure, the low concentration of triglycerides and low levels of albumin and globulin, may be caused by the type of diet administered to the animals or by peculiar characteristics of the experimental group considered. Even if the reference values for GLDH (glutamate dehydrogenase ) are not known, the results obtained from the control mice are in line with the reference ranges of other murine strains [36].

As shown by the graphical representation the values detected in the mice treated with saline and in the mice treated with inactive peptide do not differ particularly from those of mice treated with the active peptide. However, the different results show a wide and variable distribution, which is coherent with the presence of moderate individual variability. The statistical analysis (Figure 16) did not report any significant differences between the different experimental groups. The set of these data suggests that the treatment does not interfere with the operation of the main systems or vital organs and that it does not lead to the development of dysmetabolism phenomena in the animal.

Although it is not a biologically significant datum, we noted that the administration of PEP3 leads to the development of specific immunity and to the possibility of developing possible allergic/hypergic reactions. However, as previously described, neither the active nor the inactive peptide seems to induce any systemic or organ-specific damage, or to alter the haematological values as reported in the erythrogram.

### Histological analyses on the liver and brain of mice treated for 14 days with PEP3

The liver and one hemisphere of the brain of each animal were fixed in formalin and included in paraffin in order to perform the histochemical analyses. From each block of paraffin, serial sections 4µm thick, were obtained, which were stained with haematoxylin-eosin or with PAS (Periodic Acid-Shiff) staining. The use of such techniques made it possible to identify at the liver level of the animals examined the presence of widespread vacuoles localized inside the cytoplasm of the hepatocytes. However, the presence of these vacuoles does not determine the shift of the cell nucleus towards the periphery and they stained positively for PAS (Figure 17, panels A and B). Such characteristic is typical of the accumulation of glycogen, which is comparable in all the animals treated. As rodents usually eat during the night-time hours, the concentration of glycogen in the early hours of the day is higher and drops during the course of the day, therefore the comparison between the different experimental groups should consider the time of sacrifice of the animals. As shown by the haematoxylin-eosin staining, both at liver level (Figure 17, panel A) and at the CA1 region of the hippocampus (Figure 17, panel C), there are no lesions in any of the mice examined.

Subsequently, to evaluate the presence of neuro-inflammation, 5 different fields of the hippocampus were considered to verify any presence of GFAP and Iba-1 positive cells. The semi-quantitative analyses performed did not show any differences in the number of positive cells between each group, suggesting that the CPP treatment does not lead to the activation of neuro inflammatory processes (GFAP: inPEP3 31.67±1.12, PEP3 37.17±3.54; Iba-1: inPEP3 8.50±0.43, PEP3 7.33±1.63). In conclusion, treatment with the active peptide does not induce any clear morphological changes in any of the animals analysed.

### Effects of PEP3 on the ADAM10/AP2 interaction, on the synaptic localization and on the activity of the enzyme

To determine the efficacy of PEP3 following chronic treatment, the interaction between ADAM10 and the complex AP2, the synaptic localization and the activity of ADAM10 were considered.

Samples of total homogenate of the brain of the treated animals were used for the co-IP test between ADAM10 and the complex AP2 (Figure 18, panel A). The data obtained from this analysis demonstrate that chronic treatment with PEP3 can reduce the formation of the ADAM10/AP2 complex. In particular, the quantitative analysis (Figure 18, panel B) shows that the administration of PEP3 significantly reduces the interaction of ADAM10 with all the subunits of AP2 (α-adaptin PEP3 - 21.49 ± 5.20% p<0.05; β2-adaptin PEP3 -30.77 ± 7.34% p<0.05; µ2 PEP3 -23.26 ± 6.99% p<0.05).

Subsequently, all the synaptic levels of the enzyme were evaluated by analysing the TIF fraction. The Western Blot image shown in Figure 18, panel C and the related quantitative analysis (Figure 18, panel D), show a significant increase in the synaptic levels of ADAM10 in the animals treated with PEP3 (PEP3 125.60 ± 14.10% p<0.05).

The data reported by the co-IP and Western Blot analyses demonstrate that treatment with PEP3 causes a reduction of the interaction between ADAM10 and the complex AP2, determining the concomitant increase of the synaptic localization of the enzyme.

The effects of the active peptide on the activity of ADAM10 have been evaluated. Firstly the release of sAPPα was measured using an ELISA kit. As shown in Figure 19A, the total homogenate of brain of mice treated with PEP3 shows significantly higher levels of sAPPα with respect to mice treated with inactive peptide, therefore demonstrating an increase in enzyme activity (inPEP3 94.93±5.62% PEP3 121.50 ±5.99% **<0.01).

Secondly, the metabolism of another two substrates of ADAM10 was evaluated: N-Cadherin and NOTCH-1 [37,38,39]. On this point, the levels of fragments generated by the enzyme cut performed by ADAM10 on these proteins was measured. As expected, and as shown in the Western Blot image in Figure 19, panels B and D and in the respective quantitative analyses shown in Figure 19, panels C and E, treatment with PEP3 determines a significant increase in the levels of CTF, produced by the ADAM10 cut of N-Cadherin (inPEP3 90.22 ± 11.73%, PEP3 124.00 ± 5.42%, *p<0.05) and of the fragment of NOTCH-1 deriving from the activity of ADAM10 (inPEP3 101.70 ± 9.45%, PEP3 207.60 ± 23.58%, **p<0.01).

Considering all these analyses, it has been demonstrated that treatment with PEP3 interferes with the trafficking of ADAM10, determining a significant increase in the enzyme activity thereof towards its substrates.

To demonstrate the specificity of the peptide PEP3 towards the formation of the complex ADAM10/AP2, the interaction of ADAM10 with other partners was examined, in particular SAP97, as previous studies have demonstrated the fundamental role thereof on trafficking processes [20]. Through co-immunoprecipitation analysis it has been observed that the active peptide does not alter the interaction between ADAM10 and SAP97 (Figures 19F and G). Therefore, the increase in ADAM10 at synaptic level, shown in Figure 18C, can be justified with a reduction of the endocytosis processes undergone by the enzyme rather than by an increase in the forward trafficking processes.

As the complex AP2 can mediate the endocytosis process of various proteins, various co-IP experiments were performed for the purpose of investigating the specificity of the active peptide in interfering only with the ADAM10/AP2 interaction.

Samples of total homogenate of the brain of all the mice were immunoprecipitated with the antibody against the subunit β3 of the GABA-A receptor, and Western Blot analyses were performed to evaluate the interaction with the α-adaptin subunit of the complex AP2. As shown in Figure 20A, treatment with the active peptide does not alter the interaction between α-adaptin and the subunit β3 of the GABA-A receptor.

Subsequently, the interaction between the complex AP2 and two proteins that intervene in the cascade of the amyloid was studied: APP and BACE-1.

The co-IP analysis demonstrates that treatment with PEP3 does not interfere either with the interaction between APP and µ2 (Figure 20B), or with the BACE-1 and α-adaptin bond (Figure 19J - K).

Finally, the synaptic localization of BACE-1 was analysed in order to understand whether the active peptide could alter the trafficking of this enzyme. The image shown in Figure 19H and the related quantitative analysis (Figure 19I), demonstrate that the active peptide does not interfere with the synaptic localization of BACE-1. In conclusion, these results demonstrate the specificity of PEP3 and reinforce the idea that the increased ADAM10 activity towards APP is specifically determined by the alteration of the endocytosis processes of the enzyme, rather than by an alteration of the localization of the protein BACE-1.

### Effects of PEP3 on the composition of the glutamate receptors and the morphology of dendritic spines

After demonstrating that PEP3 acts by altering the synaptic localization of ADAM10 increasing the enzyme activity, it has been verified whether such treatment could cause modifications of the synaptic compositions and the morphology of the spines. From the Western Blot analyses performed by analysing the synaptic levels of the different subunits that comprise the NMDA glutamate receptor (GluN1, GluN2B and GluN2A) or AMPA (GluA1) (Figure 21), it has been observed that treatment with the active peptide does not influence the receptor composition at synaptic level. Knowing the importance of the activity of ADAM10 in the remodelling of the dendritic spines [37], and as an increase of the cut of the protein N-Cadherin has been reported, also involved in synaptic remodelling, it was verified whether treatment with PEP3 could induce modifications in the morphology of dendritic spines.

From the first description performed by Ramon y Cajal at the end of the 19th century, dendritic spines were described as an important neuron communication site and it was suggested that changes in neuron activity are reflected directly on their morphology. The spines were classified, based on their morphology, into three different categories: thin, stubby (without a well-defined "neck") and mushroom shaped [40]. To evaluate the possible effect induced by PEP3 on the spine morphology, two animals for each experimental group were perfused after 14 days of treatment and the brain was post-fixed in PFA and cut with a vibratome. To view the dendritic spines the lipophilic tracer DilDye was used. From the images acquired from the confocal microscope the number, length and width of the head of the dendritic spines of the hippocampal neurons were measured.

The morphological analysis (Figure 22) shows that treatment with PEP3 does not induce significant changes in the density, the width of the head or the distribution of the spines split into the three categories. Only the total length, in particular the length of the mushroom shaped spines, is significantly reduced after treatment with the active peptide PEP3. This result could lead to hypothesizing a negative effect on synaptic functionality, but as neither the width of the head, nor the receptor composition are shown to be altered after treatment with the active peptide, we can conclude that PEP3 does not induce a synaptic dysfunction.

Also the results obtained following the Novel Object Recognition Test (NORT) performed to evaluate the cognitive functions of the animals support the idea that PEP3 does not induce any alterations to the synaptic functionality. The animals were subjected to two days' training, to promote familiarization with the new environment. On the second day, two identical objects were placed in the arena (familiar object), and on the third day the animals were placed in the arena with the familiar object and a new object. The results indicate that both the mice treated with PEP3 and the mice treated with inPEP3 or saline solution spend more time exploring the new object with respect to the familiar object, indicating that they can discriminate between the two objects (Figure 23) This result confirms that treatment with CPP does not lead to the development of any alterations of the cognitive functions of the animals.

### EXAMPLE 6: Analysis of the efficacy of 14 days' administration of the peptide PEP3 in Alzheimer's Disease mouse models

### MATERIALS AND METHODS

Wild-type and APP/PS1 mice of 9 months were treated for 14 days with daily intraperitoneal injection of peptides at a dose of 3 nmol/g (9 animals per experimental group). The animals were maintained in an enclosure at 25 ± 2°C with an alternating light/dark cycle of 12 hours and had free access to water and food in the form of pellets (standard diet). At the end of treatment the mice were sacrificed and the brain was rapidly dissected, divided into two hemispheres and from one of these the hippocampus and cortex were isolated.

From the total hemisphere the total homogenate was obtained used for the co-immunoprecipitation experiments and the TIF fraction was purified, whereas from the hemisphere cortex the soluble fraction was obtained for measuring Aβ.

For the co-immunoprecipitation experiments, aliquots of 40-100 µg of lysate were incubated at 4°C in RIA 1X buffer (NaCl 200 mM, EDTA 10 mM, Na₂HPO₄ 10 mM, Nonidet P-40, 0.5% and 0.1% SDS) in a final volume of 150 µl with 1-2 µg of antibody. The following day the protein complex A/G (Santa Cruz Biotechnology) was added, conjugated with agarose marbles, and the incubation was continued for 2 hours. After three washes with RIA buffer the samples were loaded onto SDS-PAGE and analysed through Western Blot.

The proteins of TIF and of the soluble fraction were separated with SDS-PAGE and detected through Western Blot. The levels of Aβ were measured using an ELISA kit (IBL international).

Wild-type and APP/PS1 mice of 6 months were treated for 14 days with daily intraperitoneal injection of peptides at a dose of 3 nmol/g (9 animals per experimental group). In order to evaluate the effect of CPPs on the spatial memory (within the different experimental groups) the animals were subjected to a behavioural test, the Y-maze, which evaluates the different exploration time between a familiar arm and a new arm of the maze. The Y-maze test is based on the natural tendency of rodents to explore. When there is both the familiar arm and the new one in an environment, the animals more frequently approach and spend more time exploring the new arm with respect to the familiar arm of the maze. The test consists of two phases: familiarization and test. In the first phase each animal is placed in a Y-shaped maze with only 2 arms available for exploration and with visible cues inside and outside the maze for orientation. The animal is positioned in one arm (the starting arm) of the maze and is left to freely explore the environment for 5 minutes. Subsequently, the animal is removed from the arena and placed in the enclosure cage. After 30 minutes, the test is performed in which also the third arm of the maze is made available. The animal is positioned in the starting arm and left freely to explore the environment for 5 minutes. Both during the familiarization phase and during the test phase, the exploration times of the different arms of the maze are taken. In "normal" conditions, the animal spends more time on the recognition of the new arm with respect to the familiar one; on the contrary, i.e. when the animal has no exploration preference, the test indicates the presence of an alteration of the neuron circuits that control the spatial memory.

At the end of the test, the animals were sacrificed and the brain was rapidly dissected, divided into two hemispheres and from one of these the hippocampus and cortex were isolated.

From the total hemisphere, the total homogenate was obtained and the TIF fraction was purified to measure the levels of ADAM10 through Western Blot.

The quantization of the Western Blot analyses was performed through an image analysis program (ImageLab -BioRad). The levels of proteins were expressed as optical density (OD) related to the different measurements and were normalized on respective tubulin levels.

For the statistical analysis of the data the software GraphPad Prism 5 (GraphPad Software) was used. For the data that follow a normal distribution, the Student's T-test or the two-way ANOVA were applied followed by Tukey post-hoc test to evaluate a statistically significant difference between groups of animals treated with the inactive peptide inPEP3 or with the active peptide PEP3. The data are expressed as mean ± SEM.

### RESULTS

To understand the therapeutic efficacy of PEP3 the latter was administered for 14 days to APP/PS 1 transgenic mice, AD model [41], at the age of 9 months.

First of all, the efficacy of the treatment was evaluated by analysing the ADAM10/AP2 interaction and the synaptic levels of ADAM10.

As shown in Figures 24A and 24B, a significant reduction was observed in the ADAM10/AP2 interaction and in Figures 24 C and D an increase in the synaptic levels of ADAM10 in APP/PS1 mice treated with PEP3 with respect to APP/PS1 mice treated with inPEP3. The levels of ADAM10/AP2 interaction and the levels of ADAM10 observed in APP/PS1 mice treated with PEP3 can be compared in both experiments to the levels observed in WT mice. No modification to the total levels of ADAM10 in the different groups of animals was observed. It is interesting to note that treatment with PEP3 significantly reduces the levels of Aβ₁₋₄₂ in APP/PS 1 mice treated with PEP3 with respect to APP/PS 1 mice treated with inPEP3 (Figure 25A), indicating that treatment with PEP3 can shift the metabolism of APP in the non-amyloidogenic direction.

Treatment with PEP3 also modifies the molecular composition of the synapses. The synaptic fraction (TIF) was purified and the synaptic levels of proteins were determined through Western Blot.

A reduction in the levels of GluN2A was measured in APP/PS 1 mice treated with inPEP3 which was restored by treatment with PEP3. (Figure 25 B,C). No significant changes emerged in relation to other synaptic proteins (GluN2B, GLUA1, pS845GluA1). No modification to the total levels of proteins analysed in the different groups of animals was observed.

The efficacy of treatment with PEP3 was also evaluated in earlier stages of the disease.

The synaptic levels of ADAM10 were already reduced at the age of 6 months, when the synaptic disorders started to appear, but not at 3 months (Figure 26).

In light of these data, the cognitive dysfunctions present in mice aged 6 months were evaluated. The behavioural phenotype was determined using the Y-maze test. This test is based on the innate propensity in mice to explore new things, which in this case is expressed through the exploration of a new arm of the experimental apparatus with respect to a familiar one, already previously explored. As shown in Figure 27C, a significant reduction was observed in the ratio between the time spent exploring the new arm and the time spent exploring the familiar arm in APP/PS 1 mice with respect to wild type mice. The APP/PS 1 mice dedicate as much time exploring the new arm as the familiar one, as their ratio is about 1, and this indicates that they are not able to discriminate between the two arms.

The Y-maze test was repeated further on the same group of animals after 14 days' treatment, which consisted of a daily intraperitoneal injection of PEP3 or inPEP3, so as to evaluate the effect thereof on an AD phenotype. As shown in Figure 27D, a significant improvement in cognitive disorder in APP/PS 1 mice was observed following treatment with PEP3 with respect to those treated with inPEP3. Furthermore, the efficacy of the treatment was controlled through the analysis of the synaptic levels of ADAM10. As shown in Figure 27, panels A and B, treatment with PEP3 significantly increases the levels of ADAM10 in the synaptic fraction without modifying the expression of the enzyme.

### EXAMPLE 7: Evaluation of the cognitive abilities in APP/PS1 mice treated with PEP3

To evaluate the cognitive abilities of WT and APP/PS 1 mice of 6 months following 14 days' treatment with inPEP3 or PEP3, another behavioural test, the *Novel Object Recognition Test (NORT)* was performed. This test is based on the natural tendency of animals to explore new objects. The animals are first left free to explore two identical objects (familiar object) and, after 24 hours, they are exposed to a familiar object and to a new object. As shown in Figure 28, the WT mice treated for 14 days with PEP3 explore the new object for longer with respect to the familiar object, therefore not displaying any cognitive alterations (Figure 28, WT inPEP3 familiar object 38.81±2.76%, new object 61.19±2.76%, % exploration time, p<0.05).

The APP/PS 1 mice treated with inPEP3 instead have cognitive disorders, as they are not able to discriminate the new object from the familiar one (Figure 28, Tg inPEP3 familiar object 44.62±4.20%, new object 55.38±4.20%, % exploration time, p>0.05).

The treatment of APP/PS1 mice with PEP3 for 14 days can recover this cognitive disorder, as the animals explore the new object for significantly longer with respect to the familiar one, like WT animals (Figure 28, Tg PEP3 familiar object 40.46±3.11%, new object 59.54±3.11%, % exploration time, p<0.05).

These data demonstrate that treatment with the peptide PEP3 improves the AD phenotype of APP/PS 1 in the initial stages of the disease.

To evaluate which mechanism is at the basis of this result on behaviour, we evaluated the effect of the treatment with PEP3 on the morphology of dendritic spines. For each treatment group the number of dendritic spines at hippocampus level was counted.

The 14-day treatment of WT and APP/PS 1 animals reveals that the peptide PEP3 can significantly increase the density of dendritic spines in APP/PS 1 animals with respect to WT animals who have taken the inactive peptide.

Panel A of figure 29 shows representative images of dendritic spines in the three experimental groups; the graph in panel B shows that treatment for PEP3 significantly increases the density of dendritic spines in APP/PS 1 animals with respect to WT animals and to APP/PS 1 animals treated with inactive peptide (Figure 29, panels A-B, WT inPEP3 8.39±0.42, Tg inPEP3 9.42±0.43, Tg PEP3 9.95±0.41, no. spines/10µm, WT inPEP3 vs Tg PEP3 * p=0.02).

As shown in the graph of the panel D of the figure 29 there are no specific changes to the length of spines in APP/PS 1 animals after treatment with PEP3 (WT inPEP3 1.17±0.02 µm , Tg inPEP3 1.10±0.07 µm , Tg PEP3 1.10±0.03 µm). In the graph of panel C it is observed that the width of the spines, which is significantly reduced in APP/PS 1 animals treated with inPEP3 significantly increases in the transgenic animals treated with the active peptide (WT inPEP3 0.71±0.01 µm, Tg inPEP3 0.62±0.02 µm, Tg PEP3 0.67±0.01 µm, ****p<0.01 WT inPEP3 vs Tg inPEP3, *p=0.02 Tg inPEP3 vs Tg PEP3).

These data indicate that treatment with the peptide has a positive effect on the structure of the synapses, whose alterations represent one of the initial events of the pathology.

### REFERENCES

1. Selkoe, D. J. & Hardy, J. EMBO Mol Med 8, 595-608 (2016).
2. Carter, M. D., Simms, G. A. & Weaver, D. F. Clin. Pharmacol. Ther. 88, 475-486 (2010).
3. Colombo, A. et al. C Neurobiol. Dis. 49, 137-147 (2013).
4. Postina, R. et al. J. Clin. Invest. 113, 1456-1464 (2004).
5. Tyan, S.-H. et al. Mol. Cell. Neurosci. 51, 43-52 (2012).
6. Gralle, M., Botelho, M. G. & Wouters, F. S. Journal of Biological Chemistry 284, 15016-15025 (2009).
7. Caillé, I. et al. Development 131, 2173-2181 (2004).
8. Suh, J. et al. Neuron 80, 385-401 (2013).
9. Fol, R. et al. Acta Neuropathol. 131, 247-266 (2016).
10. Marr, R. A. & Spencer, B. J. Curr Alzheimer Res 7, 223-229 (2010).
11. Miners, J. S., Barua, N., Kehoe, P. G., Gill, S. & Love, S. J. Neuropathol. Exp. Neurol. 70, 944-959 (2011).
12. Altmeppen, H. C. et al. Mol Neurodegener 6, 36 (2011).
13. Laurén, J., Gimbel, D. A., Nygaard, H. B., Gilbert, J. W. & Strittmatter, S. M. Nature 457, 1128-1132 (2009).
14. Dohler, F. et al. Brain 137, 873-886 (2014).
15. Guillot-Sestier, M.-V., Sunyach, C., Druon, C., Scarzello, S. & Checler, F. J. Biol. Chem. 284, 35973-35986 (2009).
16. Guillot-Sestier, M.-V. et al. J. Biol. Chem. 287, 5021-5032 (2012).
17. Kuhn, P.-H. et al. Elife 5, (2016).
18. Endres, K. et al. Neurology 83, 1930-1935 (2014).
19. Lundgren, J. L. et al. Journal of Neurochemistry 135, 606-615 (2015).
20. Marcello, E. et al. J. Neurosci. 27, 1682-1691 (2007).
21. Pliássova, A. et al. Mol. Neurobiol. 53, 5710-5721 (2016).
22. Lammich, S. et al. Proc. Natl. Acad. Sci. U.S.A. 96, 3922-3927 (1999).
23. Marcello, E. et al. J. Clin. Invest. 123, 2523-2538 (2013).
24. Saraceno, C. et al. Cell Death Dis 5, e1547 (2014).
25. Marcello, E. et al. Neurobiol. Aging 33, 422.e1-10 (2012).
26. Hill, M. D. et al. Lancet Neurol 11, 942-950 (2012).
27. Stoeck, A. et al. Biochem. J. 393, 609-618 (2006).
28. Nordstedt, C., Caporaso, G. L., Thyberg, J., Gandy, S. E. & Greengard, P. Journal of Biological Chemistry 268, 608-612 (1993).
29. Carroll, R. C. et al. Proc. Natl. Acad. Sci. U.S.A. 96, 14112-14117 (1999).
30. Smith, K. R. et al. J. Neurosci. 32, 2485-2498 (2012).
31. Bonifacino, J. S. & Hurley, J. H. Retromer. Curr. Opin. Cell Biol. 20, 427-436 (2008).
32. Marcello, E., Gardoni, F., Di Luca, M. & Pérez-Otaño, I. J. Biol. Chem. 285, 10376-10384 (2010).
33. Kim, B. G., Dai, H.-N., McAtee, M., Vicini, S. & Bregman, B. S. J. Neurosci. Methods 162, 237-243 (2007).
34. Schnell, M. A., Hardy, C., Hawley, M., Propert, K. J. & Wilson, J. M. Hum. Gene Ther. 13, 155-161 (2002).
35. Mazzaccara, C. et al. PLoS ONE 3, e3772 (2008).
36. Henninger, C. et al. Toxicol. Appl. Pharmacol. 261, 66-73 (2012).
37. Malinverno, M. et al. J. Neurosci. 30, 16343-16355 (2010).
38. Reiss, K. et al. EMBO J. 24, 742-752 (2005).
39. Hartmann, D. et al. Hum. Mol. Genet. 11, 2615-2624 (2002).
40. Bourne, J. N. & Harris, K. M. Annu. Rev. Neurosci. 31, 47-67 (2008).
41. Jankowsky, J. L. et al. Hum. Mol. Genet. 13, 159-170 (2004).
42. Ittner Lars M. et al. Cell. 142, 387-397 (2010).

## Claims

1. Peptide for use in the medical field **characterized by** an amino acid sequence having the following formula (I):
YGRKKRRQRRRGGSGX₁PPX₂X₃X₄PRX₅ (I)
wherein:
X₁ and X₅ identical to or different from each other, are selected from the group consisting of L-amino acids **characterized by** apolar or polar neutral side chains;
X₂ and X₄ identical to or different from each other, are arginine or lysine;
X₃ is glutamine or asparagine.

2. Peptide for use in the medical field according to claim 1, wherein said L-amino acids **characterized by** apolar neutral side chains are selected from the group consisting of glycine, leucine, isoleucine, alanine, phenylalanine, methionine, proline, tryptophane and valine.

3. Peptide for use in the medical field according to each of claims 1-2, wherein said L-amino acids **characterized by** polar neutral side chains are selected from the group consisting of glutamine, asparagine, serine, tyrosine, threonine and cysteine.

4. Peptide for use in the medical field according to each of the preceding claims, wherein X₁ is glutamine or leucine and X₂ is arginine or lysine.

5. Peptide for use in the medical field according to claim 4, comprising or consisting of the amino acid sequence YGRKKRRQRRRGGSGQPPRQRPRG (SEQ ID NO: 1).

6. Peptide for use in the medical field according to claim 4, comprising or consisting of the amino acid sequence YGRKKRRQRRRGGSGLPPRQRPRL (SEQ ID NO: 2).

7. Peptide for use in the medical field according to claim 4, comprising or consisting of the amino acid sequence YGRKKRRQRRRGGSGQPPRQRPRE (SEQ ID NO: 3).

8. Peptide according to each of the preceding claims for use in the prevention and/or treatment of pathologies wherein an increase or accumulation of the β-amyloid peptide occurs, said pathology being Alzheimer's disease.

9. Pharmaceutical composition comprising at least one peptide according to each of claims 1-7 as the active ingredient, together with one or more pharmaceutically acceptable excipients and/or adjuvants.

10. Pharmaceutical composition according to claim 9, which is adapted to systemic administration by intravenous infusion.

11. Pharmaceutical composition according to claim 9, suitable for oral or intranasal administration.

12. Pharmaceutical composition according to each of claims 9-11, for use in the prevention and/or treatment of pathologies caused by an increase or accumulation of the β-amyloid peptide, said pathology being of Alzheimer's disease.

## Patentansprüche

1. Peptid zur Verwendung im medizinischen Bereich, **gekennzeichnet durch** eine Aminosäuresequenz mit der folgenden Formel (1):
YGRKKRRQRRRGGSGX₁PPX₂X₃X₄PRX₅ (I)
wobei:
X₁ und X₅, die gleich oder voneinander verschieden sind, aus der Gruppe ausgewählt sind, die aus L-Aminosäuren besteht, die durch unpolare oder polare neutrale Seitenketten gekennzeichnet sind;
X₂ und X₄, die gleich oder voneinander verschieden sind, sind Arginin oder Lysin;
X₃ ist Glutamin oder Asparagin.

2. Peptid zur Verwendung im medizinischen Bereich nach Anspruch 1, wobei die L-Aminosäuren, die durch unpolare neutrale Seitenketten gekennzeichnet sind, aus der Gruppe ausgewählt sind, die aus Glycin, Leucin, Isoleucin, Alanin, Phenylalanin, Methionin, Prolin, Tryptophan und Valin besteht.

3. Peptid zur Verwendung im medizinischen Bereich nach jedem der Ansprüche 1-2, wobei die L-Aminosäuren, die durch polare neutrale Seitenketten gekennzeichnet sind, aus der Gruppe ausgewählt sind, die aus Glutamin, Asparagin, Serin, Tyrosin, Threonin und Cystein besteht.

4. Peptid zur Verwendung im medizinischen Bereich nach jedem der vorhergehenden Ansprüche, wobei X₁ Glutamin oder Leucin ist und X₂ Arginin oder Lysin ist.

5. Peptid zur Verwendung im medizinischen Bereich nach Anspruch 4, umfassend oder bestehend aus der Aminosäuresequenz YGRKKRRQRRRGGSGQPPRQRPRG (SEQ ID NO: 1).

6. Peptid zur Verwendung im medizinischen Bereich nach Anspruch 4, umfassend oder bestehend aus der Aminosäuresequenz YGRKKRRQRRRGGSGLPPRQRPRL (SEQ ID NO: 2).

7. Peptid zur Verwendung im medizinischen Bereich nach Anspruch 4, umfassend oder bestehend aus der Aminosäuresequenz YGRKKRRQRRRGGSGQPPRQRPRE (SEQ ID NO: 3).

8. Peptid nach jedem der vorhergehenden Ansprüche zur Verwendung bei der Prävention und/oder Behandlung von Pathologien, bei denen eine Zunahme oder Akkumulation des β-Amyloid-Peptids auftritt, wobei die Pathologie die Alzheimer-Krankheit ist.

9. Pharmazeutische Zusammensetzung, die mindestens ein Peptid nach jedem der Ansprüche 1-7 als Wirkstoff zusammen mit einem oder mehreren pharmazeutisch verträglichen Exzipienten und/oder Adjuvantien umfasst.

10. Pharmazeutische Zusammensetzung nach Anspruch 9, die an die systemische Verabreichung durch intravenöse Infusion angepasst ist.

11. Pharmazeutische Zusammensetzung nach Anspruch 9, geeignet zur oralen oder intranasalen Verabreichung.

12. Pharmazeutische Zusammensetzung nach jedem der Ansprüche 9-11 zur Verwendung bei der Prävention und/oder Behandlung von Pathologien, die durch eine Zunahme oder Akkumulation des β-Amyloid-Peptids verursacht werden, wobei die Pathologie die Alzheimer-Krankheit ist.

## Revendications

1. Peptide utilisable dans le domaine médical **caractérisé par** une séquence d'acides aminés ayant la formule suivante (1) :
YGRKKRRQRRRGGSGX₁PPX₂X₃X₄PRX₅ (I)
dans laquelle :
X₁ et X₅ identiques ou différents l'un de l'autre, sont choisis dans le groupe constitué par les acides aminés L **caractérisés par** des chaînes latérales apolaires ou neutres polaires ;
X₂ et X₄ identiques ou différents l'un de l'autre, sont l'arginine ou la lysine ;
X₃ est la glutamine ou l'asparagine.

2. Peptide utilisable dans le domaine médical selon la revendication 1, dans lequel lesdits acides aminés L **caractérisés par** des chaînes latérales neutres apolaires sont choisis dans le groupe constitué par la glycine, la leucine, l'isoleucine, l'alanine, la phénylalanine, la méthionine, la proline, le tryptophane et la valine.

3. Peptide utilisable dans le domaine médical selon chacune des revendications 1 et 2, dans lequel lesdits acides aminés L **caractérisés par** des chaînes latérales neutres et polaires sont choisis dans le groupe constitué par la glutamine, l'asparagine, la sérine, la tyrosine, la thréonine et la cystéine.

4. Peptide utilisable dans le domaine médical selon chacune des revendications précédentes, dans lequel X₁ est la glutamine ou la leucine et X₂ est l'arginine ou la lysine.

5. Peptide utilisable dans le domaine médical selon la revendication 4, comprenant ou consistant en la séquence d'acides aminés YGRKKRRQRRGGSGQPPRQRPRG (SEQ ID NO: 1).

6. Peptide utilisable dans le domaine médical selon la revendication 4, comprenant ou consistant en la séquence d'acides aminés YGRKKRRQRRRGGSGLPPRQRPRL (SEQ ID NO: 2).

7. Peptide utilisable dans le domaine médical selon la revendication 4, comprenant ou consistant en la séquence d'acides aminés YGRKKRRQRRRGGSGQPPRQRPRE (SEQ ID NO: 3).

8. Peptide selon chacune des revendications précédentes pour une utilisation dans la prévention et/ou le traitement de pathologies dans lesquelles une augmentation ou une accumulation du peptide β-amyloïde se produit, ladite pathologie étant la maladie d'Alzheimer.

9. Composition pharmaceutique comprenant au moins un peptide selon chacune des revendications 1 à 7 en tant qu'ingrédient actif, ainsi qu'un ou plusieurs excipients et/ou adjuvants pharmaceutiquement acceptables.

10. Composition pharmaceutique selon la revendication 9, adaptée à l'administration systémique par perfusion intraveineuse.

11. Composition pharmaceutique selon la revendication 9, convenant à une administration orale ou intranasale.

12. Composition pharmaceutique selon chacune des revendications 9 à 11, pour une utilisation dans la prévention et/ou le traitement de pathologies causées par une augmentation ou une accumulation du peptide β-amyloïde, ladite pathologie étant la maladie d'Alzheimer.
